# EUROPÄISCHE PATENTSCHRIFT

(11) **EP 1 660 621 B1**
(45) Veröffentlichungstag und Bekanntmachung des Hinweises auf die Patenterteilung: **01.02.2012**
(21) Anmeldenummer: 04763956.2
(22) Anmeldetag: 10.08.2004
(51) Int. Cl.: C11D 3/50, C11D 3/16, C11D 3/37, A61K 8/89

(54) **AUF SUBSTRATOBERFLÄCHEN AUFZIEHENDE MITTEL**
AGENTS THAT ARE ABSORBED BY THE SURFACE OF SUBSTRATES
AGENTS PRISE SUR LA SURFACE DE SUBSTRATS

(30) Priorität: 19.08.2003 DE 10338070
(43) Veröffentlichungstag der Anmeldung: 31.05.2006
(73) Patentinhaber: Henkel AG & Co. KGaA, 40589 Düsseldorf (DE)
(72) Erfinder: BAUER, Andreas, 41564 Kaarst (DE); LAHN, Wolfgang, 47877 Willich (DE); FABER, Werner, 47877 Willich (DE); MEINE, Georg, 40822 Mettmann (DE)
(86) Internationale Anmeldenummer: PCT/EP2004/008938
(87) Internationale Veröffentlichungsnummer: WO 2005/019400

(56) Entgegenhaltungen:
- EP-A- 0 982 023
- EP-A- 0 982 313
- EP-A- 0 998 911
- WO-A-01/68037
- US-A- 5 422 280
- US-A- 5 858 939

## Beschreibung

Die vorliegende Erfindung betrifft Mittel, enthaltend Oligomere, Polymere oder Copolymere, die ein bestimmtes Strukturelement und zusätzlich eine auf harte und/oder weiche Substratoberflächen aufziehende, zumindest eine kationische Ladung tragende Verbindung enthalten, sowie die Verwendung solcher Mittel. Die Erfindung betrifft ferner ein Konditioniersubstrat und Verfahren zur Textilkonditionierung sowie zur Behandlung von Substratoberflächen.

Die europäischen Patentanmeldungen EP 0 982 023 A2, EP 0 998 911 A2 , EP 0 982 313 A2 und EP 0 982 022 A2 der General Electric beschreiben nicht flüchtige, polymere, copolymere oder oligomere Siloxane, in denen ein oder mehrere organische Substituenten von bestimmten Alkoholen, Aldehyden, Ketonen oder Ester abgeleitete Reste sind, die sowohl den Siloxanen als solchen als auch Zusammensetzungen, in die man die entsprechenden Siloxane einarbeitet, bestimmte vorteilhafte Eigenschaften verleihen.

Außer acht gelassen wird jedoch, wie man es erreichen kann, Mittel in Anlehnung an EP 0 998 911 A2, EP 0 982 313 A2 und EP 0 982 022 A2 bereitzustellen, die sich dadurch auszeichnen, daß die in diesen Mitteln vorhandenen Verbindungen besser auf mit dem Mittel zu behandelnde Substrate aufziehen.

Zum weiteren Stand der Technik zählt die WO 01/68037 A2, welche Kieselsäureester-Mischungen beschreibt.

Aufgabe der vorliegenden Erfindung war es von daher, Mittel bereitzustellen, die Verbindungen enthalten, die ein den Schriften EP 0 998 911 A2 , EP 0 982 313 A2 und EP 0 982 022 A2 entlehntes spezifisches Strukturelement aufweisen, welches bestimmte vorteilhafte Eigenschaften zeigt, wobei die Mittel dafür sorgen, daß diese Verbindungen besser auf damit zu behandelnde Substrate aufziehen.

Gegenstand der Erfindung ist daher ein Mittel, insbesondere Wasch- oder Reinigungsmittel oder Konditioniermittel oder kosmetisches Mittel, umfassend mindestens eine auf harte und/oder weiche Substratoberflächen aufziehende, zumindest eine kationische Ladung tragende Verbindung sowie wenigstens ein Oligomer, Polymer oder Copolymer, welches das nachfolgende Strukturelement gemäß Formel (1) wenigstens einmal enthält, 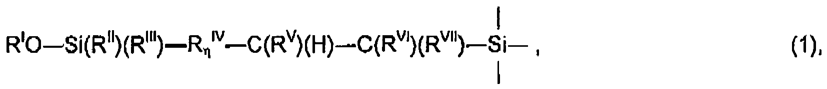 wobei R^{II}, R^{III} unabhängig voneinander jeweils für einen aliphatischen oder aromatischen, geradkettigen oder verzweigten, gesättigten oder ungesättigten, substituierten oder unsubstituierten Kohlenwasserstoffrest steht, der jeweils Heteroatome wie Sauerstoff, Stickstoff, Schwefel oder Halogene oder andere enthalten kann. Bevorzugte Reste R^{II}, R^{III} sind hier Alkyl- und/oder Alkoxyreste, beispielsweise Methyl- oder Methoxyreste. R_{η}^{IV} steht für ein Kohlenstoff-Brückenglied. Dieses Kohlenstoff-Brückenglied ist ein aliphatischer oder aromatischer, geradkettiger oder verzweigter, gesättigter oder ungesättigter, substituierter oder unsubstituierter Kohlenwasserstoffrest, der jeweils Heteroatome wie Sauerstoff, Stickstoff, Schwefel oder Halogene oder andere enthalten kann. Bevorzugt ist das Kohlenstoffbrückenglied aber ein aliphatischer Kohlenwasserstoffrest. Die Laufzahl η beträgt 0 bis 10. Dies bedeutet, daß die Gruppe -Si(R^{II})(R^{III})- aus Formel (1) auch direkt an die Gruppe -C(R^{V})(H)- aus Formel (1) gebunden sein kann, so daß in einer bevorzugten Ausführungsform kein Kohlenstoff-Brückenglied auftritt. Dies ist bei η=0 der Fall.

R^{V}, R^{VI}, R^{VII} stehen unabhängig voneinander jeweils für Wasserstoff oder einen aliphatischen oder aromatischen, geradkettigen oder verzweigten, gesättigten oder ungesättigten, substituierten oder unsubstituierten Kohlenwasserstoffrest, der jeweils Heteroatome wie Sauerstoff, Stickstoff, Schwefel oder Halogene oder andere enthalten kann. Bevorzugte Reste R^{V}, R^{VI}, R^{VII} sind Wasserstoff oder Alkylreste.

Das in der Formel (1) endständige Silicium weist an seinen verbliebenen drei Valenzen unabhängig voneinander beliebige Reste des Oligomers, Polymers oder Copolymers auf. Bevorzugt handelt es sich bei bis zu zwei dieser Reste um Alkylgruppen, insbesondere um Methylgruppen.

R^{I}O stellt entweder einen Rest dar, der eine Duftstoff-Alkoxy-Gruppe und/oder Biozid-Alkoxy-Gruppe ist, die abgeleitet ist von dem korrespondierenden Duftstoff- und/oder Biozid-Alkohol R^{I}OH, oder R^{I}O stellt einen Rest dar, der abgeleitet ist von einem enolisierbaren Duftstoff- und/oder Biozid- Ester, Keton oder Aldehyd.

Ein beliebiges Beispiel (a), in welchem R^{I}O einen Rest darstellt, der z. B. von einem enolisierbaren Duftstoff-Aldehyd abgeleitet ist, wäre

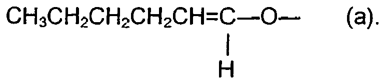

In diesem Beispiel (a) handelt es sich bei dem enolisierbaren Aldehyd um Hexanal,

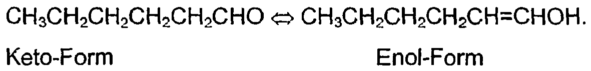

Ein beliebiges Beispiel (b) für eine erfindungsgemäße Verbindung, die das Strukturelement 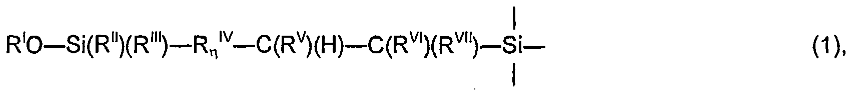 wenigstens einmal enthält, ist demnach die folgende Verbindung:

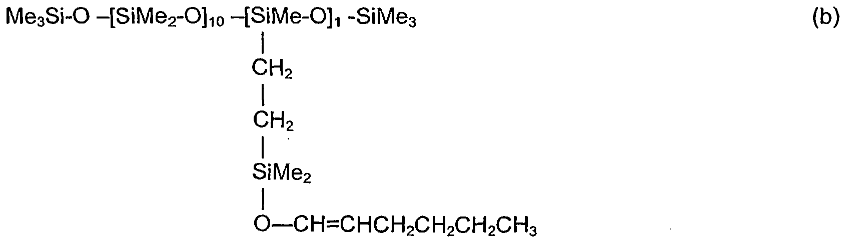

Hier ergibt sich folgende Zuordnung:

| | | |
|---|---|---|
| R^{I}O- | entspricht | CH₃CH₂CH₂CH₂CH=CH-O- |
| Si(R^{II})(R^{III})- | entspricht | SiMe₂- |
| R_{η}^{IV}- | mit η gleich 0 → | kein Brückenglied |
| C(R^{V})(H)- | entspricht | CH₂- |
| C(R^{VI})(R^{VII})- | entspricht | CH₂- |

Das endständige Silicium gemäß Formel 1 weist an seinen verbliebenen drei Valenzen unterschiedliche Reste des Oligomers auf, darunter eine Methylgrppe.
Ein weiteres beliebiges Beispiel (c) für eine erfindungsgemäße Verbindung, die das Strukturelement 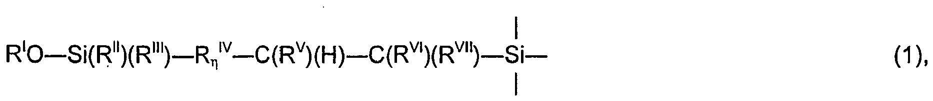 wenigstens einmal enthält, ist auch die folgende Verbindung, die sich alleine durch den Rest R^{I}O- von der vorher als Beispiel aufgeführten erfindungsgemäßen Verbindung unterscheidet:

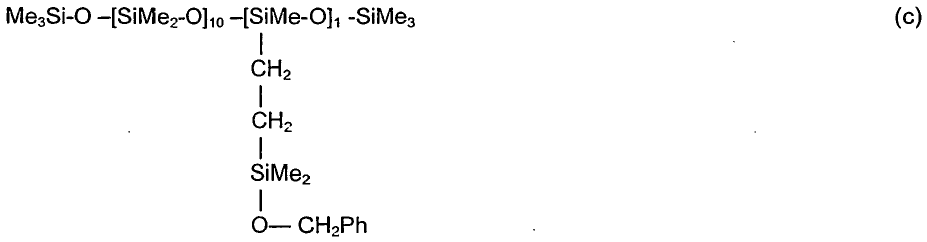

Hier ergibt sich folgende Zuordnung:

| | | |
|---|---|---|
| R^{I}O- | entspricht | PhCH₂-O- |
| Si(R^{II})(R^{III})- | entspricht | SiMe₂- |
| R_{η}^{IV}- | mit η gleich 0 → | kein Brückenglied |
| C(R^{V})(H)- | entspricht | CH₂- |
| C(R^{VI})(R^{VII})- | entspricht | CH₂- |

Das endständige Silicium gemäß Formel 1 weist an seinen verbliebenen drei Valenzen unterschiedliche Reste des Oligomers auf, darunter eine Methylgrppe.

Bei dieser Beispielverbindung gemäß (c) stellt R^{I}O einen Rest dar, der abgeleitet ist von einem Duftstoff-Alkohol, nämlich PhCH₂OH. Bei der Beispielverbindung (b) stellte R^{I}O einen Rest dar, der abgeleitet war von einem enolisierbaren Duftstoff- Aldehyd, nämlich Hexanal.

Vorteilhafte Ausführungsformen eines solchen Mittels sind duftgebende Mittel, biozide Mittel und/oder duftgebende biozide Mittel. Diese Mittel vermögen also, Duftstoffe und/oder biozide Stoffe und/oder biozid wirksame Duftstoffe abzugeben, so daß von den Mitteln als auch von den mit den Mitteln behandelten Substraten eine Duftwirkung ausgeht und/oder daß die Mittel als auch damit behandelte Substrate Biozide freizusetzen vermögen und/oder daß die Mittel als auch damit behandelte Substrate sowohl eine Duftwirkung haben als auch Biozide freizusetzen vermögen. Vorteilhafterweise erfolgt die Freisetzung der Duftstoffe bzw. der bioziden Stoffe verlangsamt, so daß also eine im Vergleich zu herkömmlichen Mitteln besonders lang anhaltende Duftwirkung und/oder biozide Wirkung verwirklicht wird.

Unter dem Begriff "Duftstoff" werden im Sinne der vorliegenden Erfindung alle diejenigen Riechstoffe bzw. Stoffe oder deren Gemische, die vom Menschen als Geruch wahrgenommen werden und beim Menschen ein Geruchsempfinden, vorzugsweise ein angenehmes Geruchsempfinden, auslösen, verstanden. Dementsprechend sind "Duftstoffalkohole" im Rahmen der vorliegenden Erfindung Duftstoffe bzw. Riechstoffe, die über freie Hydroxylgruppen verfügen, unabhängig davon wie das Molekül weiter aufgebaut ist. In Analogie dazu bezeichnen Duftstoff-Ester, -Ketone, -Aldehyde solche Duftstoffe, die entsprechend über freie Ester, Keto- oder Aldehyd-Funktionalitäten verfügen. Das impliziert, daß bestimmte Moleküle, wie z. B. Salicylsäureester im Sinne dieser Erfindung beispielsweise sowohl als Duftstoff-Alkohol als auch als Duftstoff-Ester fungieren können. Aus der großen Gruppe der Duftstoff-Alkohole, -Ester, -Ketone und -Aldehyde lassen sich bevorzugte Vertreter nennen. Solche bevorzugten Vertreter werden im weiteren Verlauf dieser Patentschrift noch genannt.

Entsprechend werden unter Biozid-Alkohol, -Aldehyd, -Ester bzw. -Keton alle Verbindungen verstanden, die die entsprechende Alkohol, Aldehyd-, Ester- bzw. Keto-Funktionalität im eben genannten Sinne besitzen, und die in der Lage sind, Keimwachstum zumindest zu hemmen. Auch hier werden im weiteren Verlauf der Patentschrift bevorzugte Vertreter genannt.
Die Begriffe "Duftstoff-Alkoxy-Gruppe" bzw. "Biozid-Alkoxy-Gruppe" erklären sich aus dem zuvor gesagten, es handelt sich hierbei um die entsprechenden Anionen der betreffenden Duftstoff-Alkohole bzw. Biozid-Alkohole, die sich durch Abstraktion eines Wasserstoffatoms ergeben.

Es wurde gefunden, daß der kombinierte Einsatz der erfindungsgemäßen Oligomere, Polymere oder Copolymere mit mindestens einer auf harte und/oder weiche Substratoberflächen aufziehenden, zumindest eine kationische Ladung tragenden Verbindung in entsprechenden Mitteln, vorzugsweise Textilbehandlungsmitteln, neben einer lang anhaltenden Produktbeduftung zu einer besonders lang anhaltenden Substratbeduftung führt, wobei die auf harte und/oder weiche Substratoberflächen aufziehende, zumindest eine kationische Ladung tragende Verbindung dazu führt, daß eine besonders vorteilhafte Substantivität der erfindungsgemäßen Oligomere, Polymere oder Copolymere auf dem behandelten Substrat erreicht wird, die sich vorzugsweise in einer besonders lang anhaltenden Duftwirkung und/oder bioziden Wirkung des Substrates manifestiert. Diese verbesserte Substantivität verleiht einem erfindungsgemäßen Mittel einen deutlichen Vorteil, da nun sichergestellt ist, daß bei der Anwendung des Mittels auf ein Substrat die Wahrscheinlichkeit erhöht ist, daß nun größere Mengen der erfindungsgemäßen Oligomere, Polymere oder Copolymere auf der Substratoberfläche fixiert werden und auch haften bleiben. Die Effizienz der Substratbeduftung oder einer bioziden Wirksamkeit wird also nachhaltig gesteigert.

Besonders vorteilhaft ist es, daß nun Mittel bereitgestellt werden können, die eine verbesserte biozide Wirkung zeigen. Dabei umfaßt der Begriff "biozide Wirkung" die üblichen Einsatzgebiete von Bioziden d. h. angefangen von einer bloßen Konservierungszwecken dienenden Wirkung bis hin zu einer direkten keimtötenden Wirkung der Biozide beispielsweise bei der Anwendung eines Mittels bei der Textilbehandlung; entscheidend für den Begriff der "bioziden Wirkung" im erfindungsgemäßen Sinne ist das Vermögen, Keimwachstum zumindest zu hemmen. Die Verbesserung der bioziden Wirkung gilt dabei sowohl im Hinblick auf eine langanhaltende Freisetzung des jeweiligen Biozids wie auch auf eine Steigerung der Substantivität von dem Biozidträger, beispielsweise einem Silikonderivat, auf einem entsprechend behandeltem Substrat. Es wird also die Effizienz des Biozides erhöht. Ferner wird es ermöglicht, Biozide über einen längeren Teitraum kontinuierlich freizusetzen und so eine besonders langanhaltende biozide Wirkung zu ermöglichen.

Die erfindungsgemäßen Vorteile der langanhaltenden Freisetzung und verbesserten Substantivität ergeben sich, wie die Anmelderin finden konnte, in gleicher Weise für Duftstoffalkohole, -ester,- ketone, -aldehyde, wie auch für Biozidalkohole, -ester,- ketone, -aldehyde. Unter den Begriff Biozidalkohole, also Verbindungen, die mindestens eine alkoholische Gruppierung aufweisen und die Keimwachstum zumindest hemmen, fallen auch Alkohole, die als Duftstoffalkohole fungieren. Insbesondere sind dies Citronellol, Eugenol, Farnesol, Thymol und Geraniol. Solche und vergleichbare biozide Duftsstoffe sind infolge ihres bifunktionellen Charakters von besonderer Vorteilhaftigkeit. Weitere Biozidalkohole sind Phenoxyethanol, 1,2-Propylenglykol, Glycerin, Zitronensäure und deren Ester, Milchsäure und deren Ester, Salicylsäure und deren Ester, 2-Benzyl-4-chlorphenol und 2,2'-Methylen- bis- (6-brom-4-chlorphenol). Nicht als Biozidalkohole im Sinne dieser Erfindung gelten die niederen Alkohole, explizit Methyl-, Ethyl- n-Propyl-, iso-Propyl-, n-Butyl-, iso-Butyl- und tert- Butylalkohol. Klassische Biozide mit Alkoholfunktionen werden dagegen erfindungsgemäß ausdrücklich als Biozidalkohole angesehen, auch wenn ihre Wirkung auf andere funktionelle Gruppen zurückzuführen ist. Beispielsweise sind hier verschiedene Bromphenole und Biphenylol sowie quartäre Ammoniumverbindungen mit mindestens einem langen Alkylrest und mindestens einem Alkylrest, der eine Hydroxy- Gruppe trägt, zu nennen.

In einer besonderen Ausführungsform zeichnen sich die erfindungsgemäßen Mittel dadurch aus, daß die erfindungsgemäßen Oligomere, Polymere oder Copolymere Biozid-Alkoxy-Gruppen beinhalten, die jeweils unabhängig voneinander abgeleitet sind von den entsprechenden Biozid-Alkoholen. Dabei können sowohl einzelne Biozidalkohole als auch Biozidalkoholgemische als auch Duftstoffalkohol- und Biozidalkohol-Gemische eingesetzt werden.

Ebenfalls und ebenso bevorzugt eingesetzt werden können Ester, Ketone und/oder Aldehyde mit biozider Wirkung im vorgenannten Sinne, wobei selbstverständlich auch gleichzeitig die Duftstoff- Ester, -Ketone und/oder -Aldehyde eine biozide Wirkung aufweisen können. Klassische Biozide, die eine Ester, Keton oder Aldehyd-Funktion aufweisen, werden hier als Biozidester, -ketone, -aldehyde angesehen, auch wenn ihre Wirkung auf andere funktionelle Gruppen zurückzuführen ist. Dabei können sowohl einzelne Biozidaldehyde, -ketone, -ester als auch entsprechende Gemische als auch entsprechende Duftstoffaldehyd, -keton, -ester und Biozidaldehyd, -keton, -ester -Gemische eingesetzt werden.

In einer weiteren bevorzugten Ausführungsform enthalten die erfindungsgemäßen Mittel das erfindungsgemäße Oligomer, Polymer oder Copolymer, vorzugsweise entsprechende Silikonderivate, in Mengen größer 0,001 Gew.-&, vorteilhafterweise von 0,002 bis 10 Gew.-%, insbesondere 0,01 bis 5 Gew.-%, besonders bevorzugt von 0,02 bis 3 und ganz besonders bevorzugt in Mengen von 0,05 bis 2 Gew.-%, jeweils bezogen auf das gesamte Mittel.

Die genauen Mengen hängen insbesondere von der Zweckbestimmung des jeweiligen Mittels ab.
So ist bei den Biozid-haltigen Oligomeren, Polymeren oder Copolymeren von Interesse, ob die Biozide lediglich eine konservierende Wirkung für das Mittel entfalten sollen, oder ob sie bei der Anwendung keimtötend wirken sollen. Dabei ist es für den Biozid- Fachmann kein Problem eine dem Anwendungszweck entsprechende Dosierung zu finden.

Sollen die Biozide zu Konservierungszwecken eingesetzt werden, so ist die Anwendung der erfindungsgemäßen Oligomere, Polymere oder Copolymere von besonderem Vorteil, da durch die vorzugsweise unter Feuchtigkeitseinfluss erfolgende langsame Hydrolyse, die Biozidkomponente über einen langen Zeitraum in geringen Mengen gleichmäßig freigesetzt wird. Beispielsweise gelingt so die langwährende Konservierung von Hautcremes mit außerordentlich niedrigen Dosen an Biozid. Insbesondere beim Einsatz in Waschmitteln kann jedoch auch die keimtötende Wirkung der Biozide bei der Anwendung des Mittels im Vordergrund stehen. Hier ergibt sich infolge des guten Aufziehverhaltens der erfindungsgemäßen Oligomere, Polymere oder Copolymere eine Wirkungssteigerung bei gleicher eingesetzter Biozidmenge.

In einer bevorzugten Ausführungsform enthält das erfindungsgemäße Mittel wenigstens ein Siliconoligomer, -polymer oder -copolymer, das bei der Hydrolyse einen duftenden und/oder bioziden Alkohol, Aldehyd, Keton oder Ester freisetzt, welcher vorzugsweise zuvor durch Umsetzung mit einem olefinischen Silan eingeführt worden ist.

So erhält man, um ein beliebiges Beispiel zu nennen, ein erfindungsgemäßes Siliconpolymer durch folgende Umsetzung eines Siloxans mit einem olefinischen Silan, das den Duftstoff trägt:

2 Ph(CH₂)₂O-Si(Me)₂-CH=CH₂ + H-[Si(Me₂)O]₂₅-H → Ph(CH₂)₂O-Si(Me)₂-(CH₂)₂-Si(Me₂)O-[Si(Me₂)O]₂₃-Si(Me₂)-(CH₂)₂- Si(Me)₂-O(CH₂)₂Ph

Bei der Hydrolyse dieses als Beispiel gewählten Reaktionsproduktes würde Ph(CH₂)₂OH freigesetzt werden.

Wenn das olefinische Silan ein Reaktionsprodukt eines duftenden und/oder bioziden Alkohols, Aldehyds, Ketons oder Esters und einem olefinischen Halosilan oder olefinischen Silikonalkoxid ist, handelt es sich um ein bevorzugtes Mittel.

So läßt sich ein erfindungsgemäßes olefinisches Silan, um ein beliebiges Beispiel zu nennen, durch Umsetzung von Phenylethylalkohol mit einem entsprechenden olefinischen Halosilan oder olefinischen Silikonalkoxid generieren, beispielsweise:

Ph(CH₂)₂OH + CH₂=CH-Si(Me)₂Cl → Ph(CH₂)₂O-Si(Me)₂-CH=CH₂ + HCl

oder beispielsweise

2 Ph(CH₂)₂OH + CH₂=CH-Si(Me)Cl₂ → {Ph(CH₂)₂O}₂Si(Me)-CH=CH₂ + 2 HCl

In einer bevorzugten Ausführungsform handelt es sich um ein olefinisches Silan nachstehender Formel (2)

(R^{VIII}O)ₐ(R^{IX}O)_{b}(R^{X}O)_{c}(R^{XI})_{d} (R^{XII})ₑSiR^{XIII} (2)

wobei R^{VIII}O, R^{IX}O und R^{X}O jeweils unabhängig voneinander Duftstoff-Alkoxy-Gruppen darstellen, die abgeleitet sind von den korrespondierenden Duftstoff-Alkoholen R^{VIII}OH, R^{IX}OH and R^{X}OH,
wobei R^{XI}, R^{XII} ausgewählt sind aus der Gruppe monovalente C₁₋₄₀ Kohlenwasserstoffreste und monovalente C₁₋₄₀Alkoxy-Reste, die jeweils aliphatisch oder aromatisch, geradkettig oder verzweigt, gesättigt oder ungesättigt, substituiert oder unsubstituiert sein können und Heteroatome wie Sauerstoff, Stickstoff, Schwefel oder Halogene oder andere enthalten können Bevorzugte Reste R^{II}, R^{III} sind Alkyl- und/oder Alkoxyreste.

R^{XIII} ist ein C₂₋₄₀ monovalenter ungesättigter Kohlenwasserstoffrest mit olefinischer Endgruppe, wobei a einen Wert von 1 - 3 hat, b, c, d, e Werte von 0-2 haben, mit der Maßgabe, daß a+b+c+d+e=3 und a, b, c, d, e ganze Zahlen sind.

Ein beliebiges Beispiel (d) für eine solche Verbindung gemäß Formel (2) wäre beispielsweise:

Ph(CH₂)₂O-Si(Me)₂-CH=CH₂ (d)

In einer weiteren bevorzugten Ausführungsform handelt es sich um ein olefinisches Silan nachstehender Formel (3):

(R^{XIV})ₐ(R^{XV})_{b}(R^{XVI})_{c}(R^{XI})_{d} (R^{XII})ₑSiR^{XII} (3)

wobei R^{XIV}, R^{XV} und R^{XVI} jeweils unabhängig voneinander die Formel (4) aufweisen,

R^{XVII}(R^{XVIII})C=C(O-)-R^{XIX} (4),

wobei R^{XVII}, R^{XVIII} und R^{XIX} unabhängig voneinander für jedes R^{XIV}, R^{XV} und R^{XVI} gewählt sind, und wobei R^{XI}, R^{XII} ausgewählt sind aus der Gruppe monovalente C₁₋₄₀ Kohlenwasserstoffreste und monovalente C₁₋₄₀ Alkoxy-Reste, die jeweils aliphatisch oder aromatisch, geradkettig oder verzweigt, gesättigt oder ungesättigt, substituiert oder unsubstituiert sein können und Heteroatome wie Sauerstoff, Stickstoff, Schwefel oder Halogene oder andere enthalten können . Bevorzugte Reste R^{II}, R^{III} sind Alkyl- und/oder Alkoxyreste.

R^{XIII} ist ein C₂₋₄₀ monovalenter ungesättigter Kohlenwasserstoffrest mit olefinischer Endgruppe ist, wobei a einen Wert von 1-3 hat, b, c, d, e Werte von 0-2 haben, mit der Einschränkung, daß a+b+c+d+e=3 und a, b, c, d, e ganze Zahlen sind, und wobei
R^{XVII}, R^{XVIII} und R^{XIX} ausgewählt sind aus der Gruppe bestehend aus Wasserstoff und monovalente C₁₋₁₀₀ Kohlenwasserstoffreste, die jeweils aliphatisch oder aromatisch, geradkettig oder verzweigt, gesättigt oder ungesättigt, substituiert oder unsubstituiert sein können und Heteroatome wie Sauerstoff, Stickstoff, Schwefel oder Halogene oder andere enthalten können . Es sei darauf hingewiesen, das die Struktur, R^{XVII}(R^{XVIII})C=C(O-)-R^{XIX} (4),
eine konjugierte Struktur ist, die mit folgender Enolat-Struktur korrespondiert,

R^{XVII}(R^{XVIII})C=C(OH)-R^{XIX} (4a),

die ein H-Atom mehr aufweist.

In der Struktur, R^{XVII}(R^{XVIII})C=C(O-)-R^{XIX} (4) symbolisiert der Strich hinter dem Sauerstoffatom rein formal ein Radikal, über welches das gesamte Strukturelement als Substituent an das betreffende olefinische Silan gebunden ist.

Ein Beispiel (e) für eine erfindungsgemäße Verbindung, gehorchend der Formel (R^{XIV})ₐ(R^{XV})_{b}(R^{XVI})_{c}(R^{XI})_{d} (R^{XII})ₑSiR^{XIII} (3), ist demnach z. B. folgende Substanz:

Me-(CH₂)₃-CH=CH-O-Si(Me)₂-CH=CH₂ (e).

In diesem Beispiel entspricht R^{XVII}(R^{XVIII})C=C(O-)-R^{XIX} (4) folgender Struktur: Me-(CH₂)₃-CH=C(O-)-H.

Die Formel, R^{XVII}(R^{XVIII})C=C(O-)-R^{XIX} (4), kann von jeder enolisierbaren Substanz mit Keto-Funktion abgeleitet werden, insbesondere von Ketonen, Aldehyden und Estern.

In einer bevorzugten Ausführungsform zeichnet sich das Mittel dadurch aus, daß R^{XIV}, R^{XV} und R^{XVI} jeweils unabhängig voneinander die Formel R^{XVII}(R^{XVIII})C=C(O-)-R^{XIX} (4) aufweisen und abgeleitet sind aus der Gruppe der nachfolgenden Aldehyde, Ketone oder Ester, die ausgewählt ist aus 3-Methyl-3(3-(1- Methylethylphenyl))propanal), 2-Methyl-3-(4-t-butylphenyl)propanal, 3- Phenylpropional, 2-Phenylpropional, Propional, Isobutyral, 2- Methylbutyral, Hexanal, Octanal, Nonanal, Decanal, 3,7-Dimethyl-1-al, p- Tolylacetaldehyd, Phenylacetaldehyd, 4-(3)(4-Methyl-3-pentenyl)-3- cyclohexen-carbaldehyd, 2,6-Dimethyl-5-heptenal, 3 ,7-Dimethyl-2,6- octadienal, trans-4-Decenal, Cyclamenaldehyd, 4-(p-Methoxyphenyl)- 2butanon, Acetophenon, 2-Pentanon, 2-Butanon, 2-Heptanon, 3- Heptanon, 2-Decanon, 3-Penten-2-on, 6-Methyl-5-hepten-2-on, Geranylacetone, 5-Methyl-alpha-ionon, 2-Acetonaphton, 2-Methyl- 3-phenylpropan-2-yl acetate, Linalylacetat, Menthanylacetat, 2-Phenylethylacetat, Tetrahydrolinalylacetat, Phenethylpropionat, Phenethylhexanoat, Butyl-acetat, Phenoxyethylisobutyrat, p-tert. -Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat, lineare Alkanale mit 8-18 C-Atomen, Citral, Citronellal, Citronellyloxy-acetaldehyd, Cyclamenaldehyd, Lilial und Bourgeonal, die Jonone, α-Isomethylionon und Methylcedrylketon.

In einer weiteren bevorzugten Ausführungsform sind die Duftstoff- und/oder Biozid-Alkoxy-Gruppen R^{VIII}O, R^{IX}O und R^{X}O jeweils abgeleitet von Duftstoff- und/oder Biozidalkoholen, ausgewählt aus der Gruppe 2-Methylbutanol, 3-Pentanol, n-Pentanol, 2-Pentanol, n-Hexanol, 2-Methylpentanol, 1-Decanol, Sandela, Nonadol, Dimetol, Thymol, 1-Heptanol, Menthol, Eugenol, Vanillin, o-Vanillin, 4-(p- Hydroxyphenyl)-2-Butanone, Syringealdehyd, Prenol, cis-3-Hexanol, trans- 3-Hexanol, cis4-Heptenol, trans-2-Octenol, trans-2-cis-6-Nonadienol, Geraniol, Nerol, Ebanol, Citronellol, Crotylalkohol, Oleylalkohol, Linalool, α-Terpineol, β-Phenethyl Alkohol, Zimtalkohol, Benzylalkohol, α-Methylbenzylalkohol, Nonylalkohol, 1-Octanol, 3-Octanol, Phenethylsalicylat, Hydrozimtalkohol, cis-6-Nonen-1-ol, trans-2-Nonen-1-ol, Methyl salicylat, cis-3-Octen-ol, Anisylalkohol, Carvacrol, Dihydrocarveol, Benzylsalicylat, Tetrahydrogeraniol, Ethylsalicylat, Ethylvanillin, Isoeugenol, Isopulegol, Laurylalkohol, Tetrahydrolinalool, 2-Phenoxyethanol, Citronellol, Eugenol, Farnesol, Thymol und Geraniol. Verbindungen dieser Art werden beispielsweise beschrieben in der EP 0 799 885, EP 0 771 785, WO 96/38528, US 5 958 870.

In einer weiteren bevorzugten Ausführungsform beträgt die Molmasse des Oligomers, Polymers oder Copolymers, die im erfindungsgemäßen Mittel enthalten sind, bis etwa 300000, vorzugsweise bis 100000, besonders bevorzugt liegen sie aber im Bereich von etwa 150 bis etwa 30000. Diese Molmassen sind deshalb vorteilhaft, weil sich hier eine besonders vorteilhaft verlängerte Duftfreisetzung und/oder Freisetzung der Biozide ergibt, verbunden mit einer überaus vorteilhaften Substantivität des Oligomers, Polymers und/oder Copolymers auf einem mit einem erfindungsgemäßen Mittel behandelten Substrat.

In einer bevorzugten Ausführungsform beträgt der Anteil des Duftstoffrestes bzw. bioziden Restes an der Gesamtmasse des Oligomers, Polymers oder Copolymers bis zu 80 Gew.-%, vorzugsweise bis zu 70 Gew.-%, insbesondere zwischen 0,001 und 60 Gew.-% liegt, jeweils bezogen auf das gesamte Mittel.

Gemäß einer weiteren bevorzugten Ausführungsform ist das Oligomer, Polymer oder Copolymer im wesentlichen unverzweigt, vorzugsweise zu mindestens 50 %, vorteilhafterweise zu mindestens 60 %, insbesondere zu mindestens 70 % linear.

In einer weiteren bevorzugten Ausführungsform gehorcht das im erfindungsgemäßen Mittel enthaltene Oligomer, Polymer oder Copolymer der nachstehenden Formel (5):

M_{f}M^{F}_{g}DₕD^{F}ᵢTⱼT^{F}ₖQₗ (5)

mit M: R^{XX}R^{XXI}R^{XXII}SiO_{1/2}; M^{F}: R^{XX}R^{XXI}R^{F}SiO_{1/2}; D: R^{XXIII}R^{XXIV}SiO_{2/2}; D^{F}: R^{XXIII}R^{F}SiO_{2/2};
T: R^{XXV}SiO_{3/2}; T^{F}: R^{F}SiO_{3/2} Q: SiO_{4/2}, wobei
R^{XX}, R^{XXI}, R^{XXII}, R^{XXIII}, R^{XXIV}, R^{XXV} jeweils unabhängig voneinander ausgewählt sind für jedes M, M^{F}, D, D^{F}, T und T^{F} aus der Gruppe der C₁₋₄₀ monovalenten, geradkettigen oder verzweigten, gesättigten oder ungesättigten Alkyl- oder Alkoxyreste oder aus der Gruppe der C₁₋₄₀ monovalenten Aryl- oder Aryloxyreste. Die vorgenannten Alkyl- , Alkoxy-, Aryl- , Aryloxyreste können substituiert oder unsubstituiert sein, sie können Heteroatome wie Sauerstoff, Stickstoff, Schwefel oder Halogene oder andere enthalten.

Die Buchstaben f , g sind positive Zahlen, h, i, j, k , l sind positive Zahlen oder gleich null, wobei wenigstens einer der h, i, j, k, l ungleich null ist und wobei zumindest einer der g, i, oder k gleich eins ist oder größer als eins;und wobei R^{F} abgeleitet ist von einem der vorgenannten und vorbeschriebenen Resten (R^{VIII}O)ₐ(R^{IX}O)_{b}(R^{X}O)_{c}(R^{XI})_{d}(R^{XII})ₑSiR^{XIII} (2) und/oder (R^{XIV})ₐ(R^{XV})_{b}(R^{XVI})_{c}(R^{XI})_{d}(R^{XII})ₑSiR^{XIII} (3) wobei dieser Rest R^{F} über ein bivalentes C₂₋₄₀ Kohlenwasserstoffbrückenglied, das abgeleitet ist von R^{XIII} (ein C₂₋₄₀ monovalenter ungesättigter Kohlenwasserstoffrest mit olefinischer Endgruppe) mit einem Si-Atom des Oligomers, Polymers oder Copolymers verbunden ist. In R^{F} ist folglich die olefinische Endgruppe nicht mehr vorhanden. Dies ist der einzige Unterschied zu den vorgenannten und vorbeschriebenen

Resten (R^{VIII}O)ₐ(R^{IX}O)_{b}(R^{X}O)_{c}(R^{XI})_{d}(R^{XII})ₑSiR^{XIII} (2)

und/oder (R^{XIV})ₐ(R^{XV})_{b}(R^{XVI})_{c}(R^{XI})_{d}(R^{XII})ₑSiR^{XIII} (3).

In einer weiteren bevorzugten Ausführungsform ist das im erfindungsgemäßen Mittel enthaltene Oligomer, Polymer oder Copolymer ausgewählt aus folgenden Formeln (6) und/oder (7): 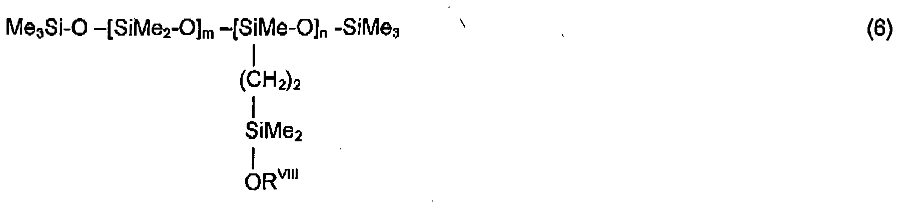 wobei OR^{VIII} für einen Duftstoff- oder Biozid-Alkoxy-Rest steht, insbesondere für eine Phenylethylalkohol-Rest. Die Buchstaben m und n haben jeweils einen positiven Wert, mit der Einschränkung daß das resultierende Silikon eine Molmasse von zumindest etwa 150 erreicht
bzw.

R^{VIII}O-SiMe₂-(CH₂)₂-[SiMe₂-O]ₚ-SiMe₂-(CH₂)₂-SiMe₂-OR^{VIII} (7)

wobei OR^{VIII} für einen Duftstoff- oder Biozid-Alkoxy-Rest steht, insbesondere für eine Phenylethylalkohol-Rest, und wobei p hat einen positiven Wert hat, mit der Einschränkung daß das resultierende Silikon eine Molmasse von zumindest etwa 150 erreicht.

Die auf harte und/oder weiche Substratoberflächen aufziehende, zumindest eine kationische Ladung tragende Verbindung ist vorzugsweise in Mengen größer 0,01 Gew.-%, vorteilhafterweise in Mengen von 0,02 bis 45 Gew.-%, insbesondere von 5 bis 40 Gew.-%, in besonders vorteilhafter Weise von 10-35 Gew.-% enthalten, jeweils bezogen auf das gesamte Mittel.

Es gibt bestimmte auf harte und/oder weiche Substratoberflächen aufziehenden Verbindungen, die in wässrigem Medium nur temporär eine positive Ladung tragen, insbesondere dann, wenn ein bestimmter pH-Bereich des Mediums vorliegt. Beispielsweise werden bestimmte stickstoffhaltige Verbindungen bei Unterschreiten definierter pH-Werte in wässrigem Medium protoniert und weisen erst dann eine positive Ladung auf.

In einer weiteren bevorzugten Ausführungsform zeichnet sich das erfindungsgemäße Mittel deshalb dadurch aus, daß es sich bei der auf harte und/oder weiche Substratoberflächen aufziehenden Verbindung um eine Verbindung handelt, die in wässrigem Medium bei pH-Werten kleiner als 4, vorzugsweise kleiner als 5, vorteilhafterweise kleiner als 6, in besonders vorteilhafter Weise kleiner 7, in ganz besonders vorteilhafter Weise kleiner als 8, in überaus vorteilhafter Weise kleiner 9, insbesondere kleiner als 10, wenigstens eine kationische Ladung aufweist, wobei der pH-Wert bei 20°C gemessen wird.

In einer weiteren bevorzugten Ausführungsform handelt es sich bei der im erfindungsgemäßen Mittel enthaltenen, auf harte und/oder weiche Substratoberflächen aufziehenden, zumindest eine kationische Ladung tragenden Verbindung um eine Verbindung, die ausgewählt ist aus der Gruppe der kationischen oder amphoteren Emulgatoren, kationischen Tenside, zwitterionischen Verbindungen, Ampholyte, Amphotenside, Betaine und/oder kationische oder amphotere Polymere.

In einer bevorzugten Ausführungsform handelt es sich bei den auf harte und/oder weiche Substratoberflächen aufziehenden, zumindest eine kationische Ladung tragende Verbindung um kationische Tenside, vorzugsweise um quartäre Ammoniumverbindungen, vorteilhafterweise um alkylierte quartäre Ammoniumverbindungen, von denen mindestens eine Alkylkette durch eine Estergruppe und/oder Amidogruppe unterbrochen ist. Der Vorteil dieser Substanzen liegt darin, daß sie nicht nur die Substantivität des Oligomers, Polymers und/oder Copolymers auf einem Substrat verbessern, sondern auch bei entsprechender Behandlung des Substrates bei diesem einen angenehm weichen Griff erzeugen.

Geeignete Beispiele sind quartäre Ammoniumverbindungen der Formeln (8) und (9), 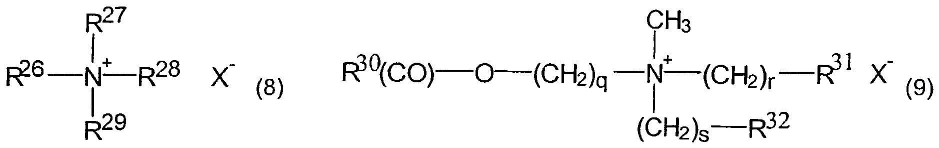 wobei in (8) R²⁶ und R²⁷ für einen acyclischen Alkylrest mit 12 bis 24 Kohlenstoffatomen, R²⁸ für einen gesättigten C₁-C₄ Alkyl- oder Hydroxyalkylrest steht, R²⁹ entweder gleich R²⁶, R²⁷ oder R²⁸ ist oder für einen aromatischen Rest steht. X- steht entweder für ein Halogenid-, Methosulfat-, Methophosphat- oder Phosphation sowie Mischungen aus diesen. Beispiele für kationische Verbindungen der Formel (8) sind Didecyldimethylammoniumchlorid, Ditalgdimethylammoniumchlorid oder Dihexadecylammoniumchlorid.

Verbindungen der Formel (9) sind sogenannte Esterquats. Esterquats zeichnen sich durch eine hervorragende biologische Abbaubarkeit aus. Hierbei steht R³⁰ für einen aliphatischen Alkylrest mit 12 bis 22 Kohlenstoffatomen mit 0, 1, 2 oder 3 Doppelbindungen; R³¹ steht für H_{b} OH oder O(CO)R^{a}, R³² steht unabhängig von R³¹ für H, OH oder O(CO)R^{b}, wobei R^{a} und R^{b} unabhängig voneinander jeweils für einen aliphatischen Alkylrest mit 12 bis 22 Kohlenstoffatomen mit 0, 1, 2 oder 3 Doppelbindungen steht. q, r und s können jeweils unabhängig voneinander den Wert 1, 2 oder 3 haben. X- kann entweder ein Halogenid-, Methosulfat-, Methophosphat- oder Phosphation sowie Mischungen aus diesen sein.

In einer bevorzugten Ausführungsform zeichnet sich das erfindungsgemäße Mittel dadurch aus, daß es sich bei der auf harte und/oder weiche Substratflächen aufziehenden Verbindung um eine quartäre Ammoniumverbindung ausgewählt aus der zuvor genannten Formel (9) handelt.

Bevorzugt sind Verbindungen, die für R³¹ die Gruppe O(CO)R^{a} und für R³⁰ und R^{a} Alkylreste mit 16 bis 18 Kohlenstoffatomen enthalten. Besonders bevorzugt sind Verbindungen, bei denen R³² zudem für OH steht. Beispiele für Verbindungen der Formel (II) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quaternierte Verbindungen der Formel (II) eingesetzt, die ungesättigte Alkylketten aufweisen, sind die Acylgruppen bevorzugt, deren korrespondierenden Fettsäuren eine Jodzahl zwischen 5 und 80, vorzugsweise zwischen 10 und 60 und insbesondere zwischen 15 und 45 aufweisen und die ein cis/trans-Isomerenverhältnis (in Gew.-%) von größer als 30 : 70, vorzugsweise größer als 50 : 50 und insbesondere größer als 70 : 30 haben. Handelsübliche Beispiele sind die von Stepan unter dem Warenzeichen Stepantex^{®} vertriebenen Methylhydroxyalkyldialkoyloxyalkylammoniummethosulfate oder die unter Dehyquart° bekannten Produkte von Cognis bzw. die unter Rewoquat^{®} bekannten Produkte von Goldschmidt-Witco. Weitere bevorzugte Verbindungen sind die Diesterquats der Formel (10), die unter dem Namen Rewoquat® W 222 LM bzw. CR 3099 erhältlich sind. Die besondere Vorteilhaftigkeit der Esterquats ergibt sich daraus, daß sie nicht nur eine gute Fixierung des Silikonderivates auf dem Substrat bewirken, sondern gleichzeitig, sofern es sich bei dem behandelten Substraten um Gewebe oder Fasern handelt, deren Weichheit fördert und ihren Griff verbessert.

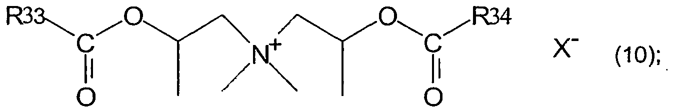

R³³ und R³⁴ stehen dabei unabhängig voneinander jeweils für einen aliphatischen Rest mit 12 bis 22 Kohlenstoffatomen mit 0, 1, 2 oder 3 Doppelbindungen.

Neben den oben beschriebenen kationischen Verbindungen können auch andere bekannte kationische Verbindungen eingesetzt werden, wie beispielsweise quartäre Imidazoliniumverbindungen der Formel (11), 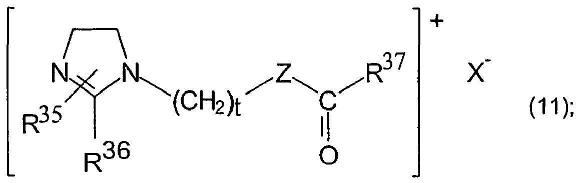 wobei R³⁵ für H oder einen gesättigten Alkylrest mit 1 bis 4 Kohlenstoffatomen, R³⁶ und R³⁷ unabhängig voneinander jeweils für einen aliphatischen, gesättigten oder ungesättigten Alkylrest mit 12 bis 18 Kohlenstoffatomen, R³⁶ alternativ auch für O(CO)R^{c} stehen kann, wobei R^{c} einen aliphatischen, gesättigten oder ungesättigten Alkylrest mit 12 bis 18 Kohlenstoffatomen bedeutet, und Z eine NH-Gruppe oder Sauerstoff bedeutet und X- ein Anion ist. t kann ganzzahlige Werte zwischen 1 und 4 annehmen.

Weitere bevorzugte quartäre, kationische Verbindungen sind durch Formel (12) beschrieben, 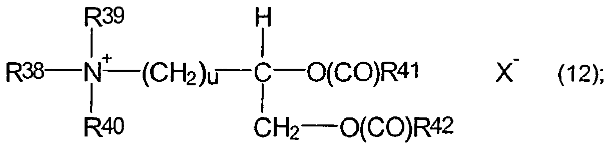 wobei R³⁸, R³⁹ und R⁴⁰ unabhängig voneinander für eine C₁₋₄-Alkyl-, Alkenyl- oder Hydroxyair kylgruppe steht, R⁴¹ und R⁴² jeweils unabhängig ausgewählt eine C₈₋₂₈-Alkylgruppe darstellt und u eine Zahl zwischen 0 und 5 ist. X⁻ ist ein passendes Anion, vorzugsweise ein Halogenid-, Methosulfat-, Methophosphat- oder Phosphation sowie Mischungen aus diesen sein.

In einer bevorzugten Ausführungsform handelt es sich bei den im erfindungsgemäßen Mittel enthaltenen, auf harte und/oder weiche Substratflächen aufziehenden Verbindung um eine quartäre Ammoniumverbindung ausgewählt aus der zuvor genannten Formel (12).

Neben den Verbindungen der Formeln (8) und (9) können auch kurzkettige, wasserlösliche, quartäre Ammoniumverbindungen eingesetzt werden, wie Trihydroxyethylmethylammoniummethosulfat oder die Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Auch protonierte Alkylaminverbindungen, die weichmachende Wirkung aufweisen, sowie die nicht quaternierten, protonierten Vorstufen der kationischen Emulgatoren sind geeignet.

Weitere erfindungsgemäß verwendbare auf harte und/oder weiche Substratoberflächen aufziehende Verbindungen stellen die quaternisierten Proteinhydrolysate dar.

Ebenfalls einsetzbar sind Verbindungen der Formel (13), 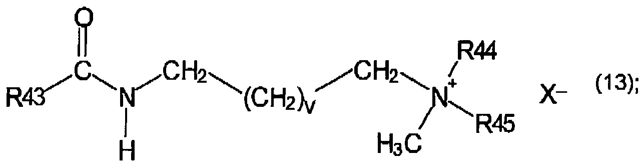 die Alkylamidoamine in ihrer nicht quaternierten oder, wie dargestellt, ihrer quaternierten Form, sein können. R⁴³ kann ein aliphatischer Alkylrest mit 12 bis 22 Kohlenstoffatomen mit 0, 1, 2 oder 3 Doppelbindungen sein. v kann Werte zwischen 0 und 5 annehmen. R⁴⁴ und R⁴⁶ stehen unabhängig voneinander jeweils für H, C₁₋₄-Alkyl oder Hydroxyalkyl. Bevorzugte Verbindungen sind Fettsäureamidoamine wie das unter der Bezeichnung Tego Amid^{®}S 18 erhältliche Stearylamidopropyldimethylamin oder das unter der Bezeichnung Stepantex^{®} X 9124 erhältliche 3-Talgamidopropyl-trimethylammonium-methosulfat, die sich neben einer guten konditionierenden Wirkung auch durch farbübertragungsinhibierende Wirkung sowie speziell durch ihre gute biologische Abbaubarkeit auszeichnen. Besonders bevorzugt sind alkylierte quaternäre Ammoniumverbindungen, von denen mindestens eine Alkylkette durch eine Estergruppe und/oder Amidogruppe unterbrochen ist, insbesondere N-Methyl-N(2-hydroxyethyl)-N,N-(ditalgacyloxyethyl)ammonium-methosulfat und/oder N-Methyl-N(2-hydroxyethyl)-N,N-(palmitoyloxyethyl)ammonium-methosulfat.

In einer bevorzugten Ausführungsform handelt es sich bei der auf harte und/oder weiche Substratoberflächen aufziehenden, zumindest eine kationische Ladung tragenden Verbindung um ein kationisches Nitril folgender Formel (14) 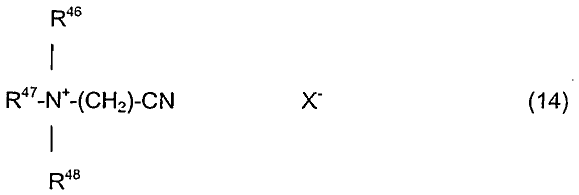 in der R⁴⁶ für -H, -CH₃, einen C₂₋₂₄-Alkyl- oder -Alkenylrest, einen substituierten C₂₋₂₄-Alkyl- oder -Alkenylrest mit mindestens einem Substituenten aus der Gruppe -Cl, -Br, -OH, -NH₂, -CN, einen Alkyl- oder Alkenylarylrest mit einer C₁₋₂₄-Alkylgruppe, oder für einen substituierten Alkyl- oder Alkenylarylrest mit einer C₁₋₂₄-Alkylgruppe und mindestens einem weiteren Substituenten am aromatischen Ring steht, R⁴⁷ und R⁴⁸ unabhängig voneinander ausgewählt sind aus -CH₂-CN, -CH₃, -CH₂-CH₃, -CH₂-CH₂-CH₃, -CH(CH₃)-CH₃, -CH₂-OH, -CH₂-CH₂-OH, -CH(OH)-CH₃, -CH₂-CH₂-CH₂-OH, -CH₂-CH(OH)-CH₃, -CH(OH)-CH₂-CH₃, -(CH₂CH₂-O)ₙH mit n = 1, 2, 3, 4, 5 oder 6 und X ein Anion ist.

Unter diese allgemeine Formel (14) fällt eine Vielzahl von kationischen Nitrilen, die alle im Rahmen der vorliegenden Erfindung einsetzbar sind. Mit besonderem Vorteil enthalten die erfindungsgemäßen Mittel dabei kationische Nitrile, in denen R⁴⁶ für Methyl, Ethyl, Propyl, Isopropyl oder einen n-Butyl, n-Hexyl, n-Octyl, n-Decyl, n-Dodecyl, n-Tetradecyl, n-Hexadecyl oder n-Octadecylrest steht. R⁴⁷ und R⁴⁸ sind vorzugsweise ausgewählt aus Methyl, Ethyl, Propyl, Isopropyl und Hydroxyethyl, wobei einer oder beide Reste vorteilhaft auch noch ein Cyanomethylenrest sein kann.

Aus Gründen der leichteren Synthese sind Verbindungen bevorzugt, in denen die Reste R⁴⁶ bis R⁴⁸ identisch sind, beispielsweise (CH₃)₃N⁺CH₂-CN X⁻, (CH₃CH₂)₃N⁺CH₂-CN X⁻, (CH₃CH₂CH₂)₃N⁺CH₂-CN X⁻, (CH₃CH(CH₃))₃N⁺CH₂-CN X⁻, oder (HO-CH₂-CH₂)₃N⁺CH₂-CN X⁻. Ein bevorzugter Gegenstand der vorliegenden Anmeldung ist daher ein erfindungsgemäßes Mittel enthaltend ein kationisches Nitril gemäß folgender Formel (15) 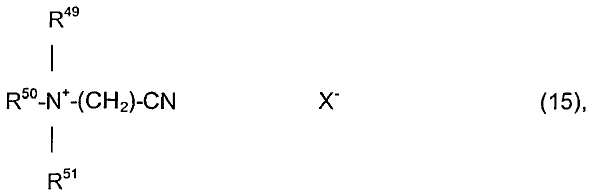 enthält, in der R⁴⁹, R⁵⁰ und R⁵¹ unabhängig voneinander ausgewählt sind aus -CH₃, -CH₂-CH₃, -CH₂-CH₂-CH₃, -CH(CH₃)-CH₃, wobei R⁴⁹ zusätzlich auch -H sein kann und X ein Anion ist, wobei vorzugsweise R⁵⁰ = R⁵¹ = -CH₃ und insbesondere R⁴⁹ = R⁵⁰ = R⁵¹ = -CH₃ gilt und Verbindungen der Formeln (CH₃)₃N⁽⁺⁾CH₂-CN X⁻, (CH₃CH₂)₃N⁽⁺⁾CH₂-CN X⁻ , (CH₃CH₂CH₂)₃N⁽⁺⁾CH₂-CN X⁻, (CH₃CH(CH₃))₃N⁽⁺⁾CH₂-CN X⁻, oder (HO-CH₂-CH₂)₃N⁽⁺⁾CH₂-CN X- besonders bevorzugt sind.

Erfindungsgemäße Mittel, die ein kationisches Nitril der beiden vorgenannten Formeln, vorzugsweise der letztgenannten Formel, besonders bevorzugt der Formel (CH₃)₃N⁽⁺⁾CH₂-CN X⁻ enthalten, wobei X- für ein Anion steht, das aus der Gruppe Chlorid, Bromid, lodid, Hydrogensulfat, Methosulfat, Laurylsulfat, Dodecylbenzolsulfonat, p-Toluolsulfonat (Tosylat), Cumolsulfonat oder Xylolsulfonat oder deren Mischungen ausgewählt ist, sind besonders bevorzugt.

Selbstverständlich kann ein erfindungsgemäßes Mittel mehrere kationische Nitrile der zuvor beschriebenen Struktur enthalten. Technisch realisierbar und im Rahmen der vorliegenden Anmeldung bevorzugt sind Mittel, die zwei, drei, vier oder fünf verschiedene kationische Nitrile enthalten.

Ein besonderer Vorteil des Einsatzes der erfindungsgemäßen Nitrilquats ergibt sich aus deren Funktion als Bleichaktivator.

Der Gewichtsanteil der kationischen Nitrile am Gesamtgewicht des erfindungsgemäßen Mittels kann variieren, wobei der Gewichtsanteil des kationischen Nitrils. bis zu 60 Gew.-% betragen kann. Im Rahmen der vorliegenden Anmeldung werden jedoch solche erfindungsgemäßen Mittel bevorzugt, die einen Gewichtsanteil an kationischem Nitril zwischen 0,01 bis 40 Gew.-%, vorzugsweise von 0,1 bis 32 Gew.-%, bevorzugt von 0,2 bis 28 Gew.-%, besonders bevorzugt von 0,5 bis 24 Gew.-% und insbesondere von 1,0 bis 20 Gew.-%, jeweils bezogen auf das Gesamtgewicht des Mittels, aufweisen.

In einer bevorzugten Ausführungsform handelt es sich bei der auf harte und/oder weiche Substratoberflächen aufziehenden Verbindung um ein Polymer, welches vorzugsweise mindestens eine positive Ladung trägt, insbesondere um kationische oder amphothere Polymere, beispielsweise um kationische oder amphotere Zucker- oder Stärkderivate oder um kationische oder amphotere Cellulosederivate.

Zu den geeigneten kationischen Polymeren zählen die Polyquaternium-Polymere, wie sie im CTFA Cosmetic Ingredient Dictionary (The Cosmetic, Toiletry und Fragrance, Inc., 1997), insbesondere die auch als Merquats bezeichneten Polyquaternium-6-, Polyquaternium-7-, Polyquaternium-10-Polymere (Ucare Polymer IR 400; Arnerchol), Polyquaternium-4-Copolymere, wie Pfropfcopolymere mit einem Cellulosegerüst und quartären Ammoniumgruppen, die über Allyldimethylammoniumchlorid gebunden sind, kationische Cellulosederivate, wie kationisches Guar, wie Guar-hydroxypropyltriammoniumchlorid, und ähnliche quaternierte Guar-Derivate (z. B. Cosmedia Guar, Hersteller: Cognis GmbH), kationische quartäre Zuckerderivate (kationische Alkylpolyglucoside), z. B. das Handelsprodukt Glucquat^{®}100, gemäß CTFA-Nomenklatur ein "Lauryl Methyl Gluceth-10 Hydroxypropyl Dimonium Chloride", Copolymere von PVP und Dimethylaminomethacrylat, Copolymere von Vinylimidazol und Vinylpyrrolidon, Aminosilicon-polymere und Copolymere.

Ebenfalls einsetzbar sind polyquaternierte Polymere (z. B. Luviquat Care von BASF) und auch kationische Biopolymere auf Chitinbasis und deren Derivate, beispielsweise das unter der Handelsbezeichnung Chitosan° (Hersteller: Cognis) erhältliche Polymer.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) Abil^{®}-Quat 3270 und 3272 (Hersteller: Goldschmidt-Rewo; diquartäre Polydimethylsiloxane, Quaternium-80), sowie Siliconquat Rewoquat^{®} SQ 1 (Tegopren^{®} 6922, Hersteller: Goldschmidt-Rewo).

Besonders bevorzugt kationische oder amphotere Polymere enthalten mindestens eine ethylenisch ungesättigte Monomereinheit der allgemeinen Formel (16)

R⁵²(R⁵³)C=C(R⁵⁴)R⁵⁵ (16)

in der R⁵² bis R⁵⁵ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, - OH oder -COOH substituierte Alkyl- oder Alkenylreste wie vorstehend definiert, eine heteroatomare Gruppe mit mindestens einer positiv gelandenen Gruppe, einem quaternisierten Stickstoffatom oder zumindest einer Amingruppe mit einer postiven Ladung im pH-Bereich zwischen 2 und 11 oder für -COOH oder -COOR^{d} steht, wobei R^{d} ein gesättigter oder ungesättigter, geradkettigter oder verzweigter Kohlenwasserstoffrest mit 1 bis 12 Kohlenstoffatomen ist.

Beispiele für die vorgenannten (unpolmyerisierten) Monomereinheiten sind Diallylamin, Methyldiallylamin, Dimethyldimethylammoniumsalze, Acrylamidopropyl(trimethyl)ammoniumsalze (R⁵², R⁵³, und R⁵⁴, = H, R⁵⁵ = C(O)NH(CH₂)₂N⁺(CH₃)₃ X⁻), Methacrylamidopropyl(trimethyl)ammoniumsalze (R⁵² und R⁵³ = H, R⁵⁴ = CH₃ H, R⁵⁵ = C(O)NH(CH₂)₂N⁺(CH₃)₃ X-).

Besonders bevorzugt als Bestandteil der amphoteren Polymere werden ungesättigte Carbonsäuren der allgemeinen Formel (17)

R⁵⁶(R⁵⁷)C=C(R⁵⁸)COOH (17)

eingesetzt, in der R⁵⁶ bis R⁵⁸ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR^{e} steht, wobei R^{e} ein gesättigter oder ungesättigter, geradkettigter oder verzweigter Kohlenwasserstoffrest mit 1 bis 12 Kohlenstoffatomen ist.

Besonders bevorzugte amphotere Polymere enthalten als Monomereinheiten Derivate des Diallylamins, insbesondere Dimethyldiallylammoniumsalz undloder Methacrylamidopropyl(trimethyl)-ammoniumsalz, vorzugsweise in Form des Chlorids, Bromids, lodids, Hydroxids, Phosphats, Sulfats, Hydrosulfats, Ethylsulfasts, Methylsulfats, Mesylats, Tosylats, Formiats oder Acetats in Kombination mit Monomereinheiten aus der Gruppe der ethylenisch ungesättigten Carbonsäuren.

In einer weiteren bevorzugten Ausführungsform handelt es sich bei der auf harte und/oder weiche Substratoberflächen aufziehende, zumindest eine kationische Ladung tragende Verbindung um eine zwitterionische Verbindung. Eine zwitterionische Verbindung zeichnet sich dadurch aus, daß im gleichen Molekül sowohl eine Gruppe mit positiver Ladung als auch eine mit negativer Ladung auftreten. Zu der Gruppe der zwitterionischen Verbindungen zählen beispielsweise auch die Betaine. Betaine im engeren Sinne sind Verbindungen die eine A-tomgruppierung R₃N⁺-CH₂-COO⁻ aufweisen, als Betaine im weiteren Sinne können aber auch andere zwitterionische Verbindungen aufgefasst werden, in denen sich beispielsweise die positive Ladung am N oder P-Atom und die negative Ladung sich formal am O, S, B oder C-Atom befindet.

In einer weiteren bevorzugten Ausführungsform handelt es sich bei der auf harte und/oder weiche Substratoberflächen aufziehende, zumindest eine kationische Ladung tragende Verbindung um eine Ampholyten. Ampholyten sind chemische Verbindungen, die in wässrigen Medien ionisieren können und dabei in Abhängigkeit des pH-Wertes des Mediums einen anionischen oder kationischen Charakter erhalten, vorzugsweise können Ampholyte sowohl Protonen aufnehmen als auch abspalten können, also in saurer Lösung Kationen, in alkalischer Lösung Anionen bilden.

In einer weiteren bevorzugten Ausführungsform handelt es sich bei der auf harte und/oder weiche Substratoberflächen aufziehende, zumindest eine kationische Ladung tragende Verbindung um eine zwitterionische Verbindung nachstehender Formel (18) 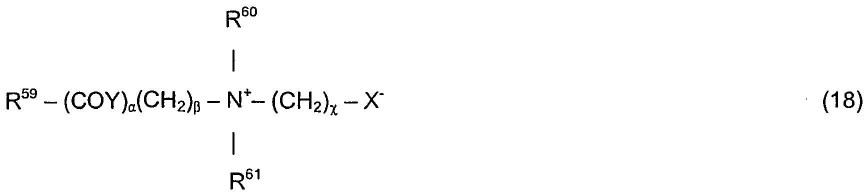 wobei das R⁵⁹ für eine C₆₋₂₈-Alkyl- oder Alkenylgruppe steht; R⁶⁰ und R⁶¹ sind jeweils, unabhängig voneinander, C₁₋₄ Alkyl-Gruppen; α, steht für die Zahl 0 oder 1, β und χ sind jeweils, unabhängig voneinander, aus ganzen Zahlen von 1 bis 4 ausgewählt; Y ist Sauerstoff oder Stickstoff; X ist ein kompatibles Anion.
Vorzugweise handelt es sich um ein Alkylamidoalkylendimethylcarbonsäure-Betain mit der nachstehenden Formel (19): 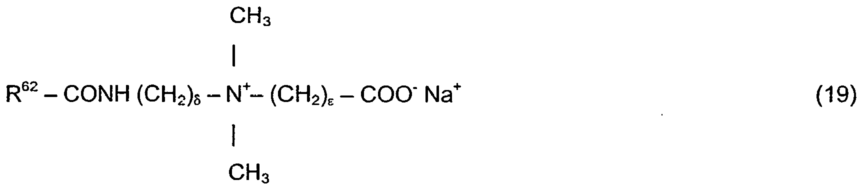 wobei δ und ε, unabhängig voneinander, ganze Zahlen von 1-4 sind, vorzugsweise ist b gleich 2 oder 3 und c gleich 2 oder 3 und R⁶² steht für eine C₁₀₋₁₈-Alkylkette oder Mischungen davon.

In einer bevorzugten Ausführungsform liegt das erfindungsgemäße Mittel in fester, dispergierter, pulvriger, granulärer oder gepresster Form vor. Liegt das Mittel in gepresster Form vor, dann insbesondere in Form von Tabletten, die aus einer einzigen oder mehreren Phasen bestehen.

Es kann aber auch zweckdienlich im Hinblick auf die Anwendung des Mittels sein, das Mittel in fluider Form bereitzustellen.

In einer bevorzugten Ausführungsform liegt das Mittel daher in gelförmiger oder flüssiger Form vor, insbesondere emulgiert, wobei vorzugsweise bis zu 95 Gew.-%, bevorzugt 20 bis 90 Gew.-%, weiter bevorzugt 50-80 Gew.-% eines oder mehrerer Lösungsmittel enthalten ist.

Es kann jedoch aus anwendungstechnischer Sicht erwünscht sein, daß erfindungsgemäße, vorzugsweise fluide Mittel bereitgestellt werden, die wenig bis kein Wasser enthalten. In einer weiteren bevorzugten Ausführungsform liegt das Mittel daher in nichtwäßriger Form vor. Unter nichtwäßriger Form im Rahmen dieser Erfindung werden Wassergehalte unter 15 Gew.-%, bezogen auf das Mittel, verstanden, bevorzugt Wassergehalte unter 10 Gew.%, besonders bevorzugt unter 8 Gew.-%, Von besonderer Bedeutung sind Wassergehalte kleiner 6 Gew.%, insbesondere sind aber Wassergehalte zwischen 2 und 0,001 Gew.-%, bezogen auf das Mittel, bevorzugt.

Der Vorteil einer Reduktion des Wasseranteils im Mittel liegt darin, daß die Inhaltsstoffe des Mittels bei der Applikation in konzentrierter Form eingesetzt werden können, sowie daß die Mittel besser verarbeitbar, beispielsweise emulgierbar sind.

Bei den erfindungsgemäßen flüssigen Mitteln handelt es sich vorzugsweise um Flüssigwaschmittel, flüssige Geschirrspülmittel, Reinigungsgele, flüssige Textilbehandlungsmittel oder flüssige Haut- und/oder Haarreinigungs- und/oder -pflegemittel.

Die erfindungsgemäßen Mittel können neben den erfindungsgemäßen Bestandteilen in Form des Oligomers, Polymers oder Copolymers und der auf harte und/oder weiche Substratoberflächen aufziehenden, zumindest eine kationische Ladung tragende Verbindung beliebige im Zusammenhang mit dem vorgesehenen Verwendungszweck relevante Inhalts- und Aktivstoffe enthalten.

Dabei bereitet es dem jeweiligen Fachmann keine Schwierigkeiten, die jeweiligen, zusätzlich erforderlichen Inhaltsstoffe auszuwählen.

Besondere Bedeutung im Hinblick auf die erfindungsgemäßen Mittel ist neben den kosmetischen Formulierungen zur Reinigung und Pflege des Körpers dem Gesamtkomplex der Wasch- und Reinigungsmittel zuzumessen. Daher werden nachfolgend wichtige Inhaltsstoffe aufgeführt, die in den erfindungsgemäßen Mitteln zusätzlich enthalten sein können, vorzugsweise dann, wenn diese Mittel den Gesamtkomplex der Wasch- und Reinigungsmittel betreffen. In diesen Mitteln können prinzipiell alle im Zusammenhang mit einem Wasch- oder Reinigungsvorgang relevanten Substanzen enthalten sein. Darunter fallen Aktivstoffe wie Tenside (beispielsweise anionische, nichtionische Tenside), Buildersubstanzen (anorganische und organische Buildersubstanzen), Bleichmittel (wie beispielsweise Peroxo-Bleichmittel und Chlor- Bleichmittel), Bleichaktivatoren, Bleichstabilisatoren, Bleichkatalysatoren, Enzyme, spezielle Polymere (beispielsweise solche mit Cobuilder-Eigenschaften), Vergrauungsinhibitoren. Es können auch Waschhilfsmittel und Reinigungshilfsmittel enthalten sein. Beispiele für diese sind optische Aufheller, UV-Schutzsubstanzen sowie sog. Soil Repellents, also Polymere, die einer Wiederanschmutzung von Fasern oder harten Oberflächen entgegenwirken. Weiterhin kann das erfindungsgemäße Mittel ein oder mehrere übliche Hilfs- und Zusatzstoffe, insbesondere ausgewählt aus der Gruppe der Elektrolyte, Farbmittel, Riechstoffe, Duftstoffe, Parfumträger, pH-Stellmittel, Komplexbildner, Fluoreszenzmittel, Schauminhibitoren, Vergrauungsinhibitoren, Knitterschutzmittel, Antioxidantien, Antistatika, Bügelhilfsmittel, UV-Absorber, Antiredepositionsmittel, Germizide, antimikrobielle Wirkstoffe, Fungizide, Viskositätsregulatoren, Perlglanzgeber, Farbübertragungsinhibitoren, Einlaufverhinderer, Korrosionsinhibitoren, Konservierungsmittel, Weichmacher, Weichspüler, Proteinhydrolysate, Phobier- und Imprägniermittel, nichtwässrige Lösungsmittel, Hydrotrope, Silikonöle sowie Quell- und Schiebefestmittel sowie quartären Ammoniumverbindungen gegebenenfalls mit Esterbindungen enthalten.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von Sulfofettsäuren (Estersulfonate), z.B. die sulfonierten Methylester der hydrierten Kokos-, Palmkern- oder Talgfettsäuren geeignet.

Weitere geeignete Aniontenside sind sulfierte Fettsäureglycerinester. Unter Fettsäuregiycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure. Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

In einer bevorzugten Ausführungsform enthält das erfindungsgemäße Mittel ein oder mehrere Sulfosuccinate, Sulfosuccinamate und/oder Sulfosuccinamide, vorzugsweise Sulfosuccinate und/oder Sulfosuccinamate, insbesondere Sulfosuccinate, in einer Menge von üblicherweise 0,05 bis 15 Gew.-%, vorzugsweise 0,1 bis 10 Gew.-%, insbesondere 0,3 bis 6 Gew.-%, besonders bevorzugt 0,5 bis 3 Gew.%, äußerst bevorzugt 0,7 bis 2 Gew.-%, beispielsweise 0,75 oder 1,5 Gew.-%.

Als weitere Komponente können die erfindungsgemäßen Mittel gegebenenfalls ein oder mehrere nichtionische Tenside enthalten.

Als nichtionische Tenside werden vorzugsweise alkoxylierte, vorteilhafterweise ethoxylierte und/oder propoxylierte, insbesondere primäre Alkohole mit vorzugsweise 8 bis 18 C-Atomen und durchschnittlich 1 bis 12 Mol Ethylenoxid (EO) und/oder 1 bis 10 Mol Propylenoxid (PO) pro Mol Alkohol, eingesetzt. Besonders bevorzugt sind C₈-C₁₆-Alkoholalkoxylate, vorteilhafterweise ethoxylierte und/oder propoxylierte C₁₀-C₁₅-Alkoholalkoxylate, insbesondere C₁₂-C₁₄-Alkoholalkoxylate, mit einem Ethoxylierungsgrad zwischen 2 und 10, vorzugsweise zwischen 3 und 8, und/oder einem Propoxylierungsgrad zwischen 1 und 6, vorzugsweise zwischen 1,5 und 5. Der Alkoholrest kann vorzugsweise linear oder besonders bevorzugt in 2-Stellung methylverzweigt sein bzw. lineare und methylverzweigte Reste im Gemisch enthalten, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂-₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungs- und Propoxylierungsgrade stellen statistische Mittelwerte dar, die für ein spezielles Produkt eine ganze oder eine gebrochene Zahl sein können. Bevorzugte Alkoholethoxylate und -propoxylate weisen eine eingeengte Homologenverteilung auf (narrow range ethoxylates/propoxylates, NRE/NRP). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Bevorzugte nichtionische Tenside sind ein oder mehrere mit Ethylen- (EO) und/oder Propylenoxid (PO) alkoxylierte, unverzweigte oder verzweigte, gesättigte oder ungesättigte C₁₀₋₂₂-Alkohole mit einem Alkoxylierungsgrad bis zu 30, vorzugsweise ethoxylierte C₁₀₋₁₈-Fettalkohole mit einem Ethoxylierungsgrad von weniger als 30, bevorzugt 1 bis 20, insbesondere 1 bis 12, besonders bevorzugt 1 bis 8, äußerst bevorzugt 2 bis 5, beispielsweise C₁₂₋₁₄-Fettalkoholethoxylate mit 2, 3 oder 4 EO oder eine Mischung von der C₁₂₋₁₄-Fettalkoholethoxylate mit 3 und 4 EO im Gewichtsverhältnis von 1 zu 1 oder Isotridecylalkoholethoxylat mit 5, 8 oder 12 EO, wie sie beispielsweise in der DE 40 14 055 C2 (*Grillo-Werte*), auf die in dieser Hinsicht Bezug genommen und deren Inhalt hiermit in diese Anmeldung aufgenommen wird, beschrieben werden.

Die nichtionischen Tenside können üblicherweise in Mengen bis zu 50 Gew.%, vorzugsweise von 0,1 bis 40 Gew.%, besonders bevorzugt von 0,5 bis 30 und insbesondere von 2 bis 25 Gew.-%, jeweils bezogen auf das gesamte Mittel, vorlieben.

In einer bevorzugten Ausführungsform liegen die erfindungsgemäßen Mittel in flüssiger Form vor. Zum Erreichen einer flüssigen Konsistenz kann der Einsatz sowohl flüssiger organischer Lösungsmittel, wie auch der von Wasser angezeigt sein. Die erfindungsgemäßen Mittel enthalten daher gegebenenfalls Lösungsmittel.

Neben den waschaktiven Substanzen sind Builder und Co-Builder die wichtigsten Inhaltsstoffe von Wasch- und Reinigungsmitteln. In den erfindungsgemäßen Mitteln können üblicherweise in Wasch-, Spol- und Reinigungsmitteln eingesetzte Builder enthalten sein, insbesondere also Zeolithe, Silicate, Carbonate, organische Cobuilder und - wo keine ökologischen Vorurteile gegen ihren Einsatz bestehen - auch die Phosphate.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern deren Einsatz aus ökologische Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- und Reinigungsmittel-Portionen gemäß der Erfindung. Insbesondere sind in diesem Zusammenhang Citronensäure, Bernsteinsäure, Glutarsäure, Adipinsäure, Gluconsäure und beliebige Mischungen von diesen zu nennen.

Als Builder sind weiter polymere Polycarboxylate geeignet. Dies sind beispielsweise die Alkalimetalisalze der Polyacrylsäure oder der Polymethacrylsäure, beispielsweise solche mit einer relativen Molekülmasse von 500 bis 70.000 g/mol.

Bei den für polymere Polycarboxylate angegebenen Molmassen handelt es sich im Rahmen der vorliegenden Erfindung um gewichtsmittlere Molmassen M_{W} der jeweiligen Säureform, die grundsätzlich mittels Gelpermeationschromatographie (GPC) bestimmt wurden, wobei ein UV- Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsäuren gemessenen Molmassen sind in der Regel deutlich höher als die im Rahmen der vorliegenden Erfindung angegebenen Molmassen.

Geeignete Polymere sind insbesondere Polyacrylate, die bevorzugt eine Molmasse von 2.000 bis 20.000 g/mol aufweisen. Aufgrund ihrer überlegenen Löslichkeit können aus dieser Gruppe wiederum die kurzkettigen Polyacrylate bevorzugt sein, die Molmassen von 2.000 bis 10. 000 g/mol, besonders bevorzugt von 3.000 bis 5.000 g/mol, aufweisen.

Geeignet sind weiterhin copolymere Polycarboxylate, insbesondere solche der Acrylsäure mit Methacrylsäure oder der Acrylsäure oder Methacrylsäure mit Maleinsäure. Als besonders geeignet haben sich Copolymere der Acrylsäure mit Maleinsäure erwiesen, die 50 bis 90 Gew.% Acrylsäure und 50 bis 10 Gew.-% Maleinsäure enthalten. Ihre relative Molmasse, bezogen auf freie Säuren, beträgt im allgemeinen 2.000 bis 70. 000 g/mol, vorzugsweise 20.000 bis 50.000 g/mol und insbesondere 30.000 bis 40.000 g/mol.

Die (co-)polymeren Polycarboxylate können entweder als Pulver oder als wäßrige Lösung eingesetzt werden. Der Gehalt der erfindungsgemäßen Mittel an (co-) polymeren Polycarboxylaten beträgt vorzugsweise 0,5 bis 20 Gew.%, insbesondere 3 bis 10 Gew.-%.

Auch Oxydisuccinate und andere Derivate von Disuccinaten, vorzugsweise Ethylendiamindisuccinat sind weitere geeignete Co-Builder.

Eine weitere Substanzklasse mit Co-Builder-Eigenschaften stellen die Phosphonate dar.

Darüber hinaus können alle Verbindungen, die in der Lage sind, Komplexe mit Erdalkalimetallionen zu bilden, als Co-Builder eingesetzt werden.

In einer bevorzugten Ausführungsform kann das erfindungsgemäße Mittel gegebenenfalls zusätzlich ein oder mehrere Komplexbildner enthalten.

Komplexbildner (INCI Chelating Agents), auch Sequestriermittel genannt, sind Inhaltsstoffe, die Metallionen zu komplexieren und inaktivieren vermögen, um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert die oxidative Zersetzung der fertigen Mittel.

Ein besonders bevorzugter Komplexbildner ist die Etidronsäure (1-Hydroxyethyliden-1,1-diphosphonsäure, 1-Hydroxyethyan-1,1-diphosphonsäure, HEDP, Acetophosphonsäure, INCI Etidronic Acid) einschließlich ihrer Salze. In einer bevorzugten Ausführungsform enthält das erfindungsgemäße Mittel demgemäß als Komplexbildner Etidronsäure und/oder eines oder mehrere ihrer Salze.

Das erfindungsgemäße Mittel enthält Komplexbildner in einer Menge von üblicherweise 0 bis 20 Gew.-%, vorzugsweise 0,1 bis 15 Gew.-%, insbesondere 0,5 bis 10 Gew.-%, besonders bevorzugt 1 bis 8 Gew.%, äußerst bevorzugt 1,5 bis 6 Gew.-%, beispielsweise 1,5, 2,1, 3 oder 4,2 Gew.-%.

In einer weiteren Ausführungsform enthält das erfindungsgemäße Mittel gegebenenfalls ein oder mehrere Viskositätsregulatoren, die vorzugsweise als Verdicker fungieren.

Die Viskosität der Mittel kann mit üblichen Standardmethoden (beispielsweise Brookfield-Viskosimeter RVD-VII bei 20 U/min und 20°C, Spindel 3) gemessen werden und liegt vorzugsweise im Bereich von 10 bis 5000 mPas. Bevorzugte flüssige bis gelförmige Mittel haben Viskositäten von 20 bis 4000 mPas, wobei Werte zwischen 40 und 2000 mPas besonders bevorzugt sind.

Geeignete Verdicker sind anorganische oder polymere organische Verbindungen. Es können auch Gemische aus mehreren Verdickern eingesetzt werden.

In einer weiteren bevorzugten Ausführungsform enthält das erfindungsgemäße Mittel gegebenenfalls ein oder mehrere Enzyme.

Als Enzyme kommen insbesondere solche aus der Klassen der Hydrolasen wie der Proteasen, Esterasen, Lipase bzw. lipolytisch wirkende Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen wie protein-, fett- oder stärkehaltigen Verfleckungen und Vergrauungen bei. Cellulasen und andere Glykosylhydrolasen können darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipasehaltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können als Formkörper an Trägerstoffe adsorbiert oder gecoated eingebettet sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,12 bis etwa 2 Gew.-% betragen.

Die Mittel können gegebenenfalls zusätzliche Bleichmittel enthalten. Unter den als Bleichmittel dienenden, in Wasser H₂O₂ liefernden Verbindungen haben das Natriumpercarbonat, das Natriurrtperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphtoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Die Bleichmittel können gecoated sein, um sie gegen vorzeitige Zersetzung zu schützen.

Farbstoffe können im erfindungsgemäßen Mittel eingesetzt werden, wobei die Menge an einem oder mehreren Farbstoffen so gering zu wählen ist, daß nach der Anwendung des Mittels keine sichtbaren Rückstände verbleiben. Vorzugsweise ist das erfindungsgemäße Mittel aber frei von Farbstoffen.

Weiterhin können die Mittel gegebenenfalls Knitterschutzmittel btw. -reduktionsmittel enthalten. Hierzu zählen beispielsweise synthetische Produkte auf der Basis von Fettsäuren, Fettsäureestern. Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Die erfindungsgemäßen Mittel lassen eine Vielzahl von Verwendungsmöglichkeiten zu. Eine bevorzugte Ausführungsform stellt die Verwendung eines erfindungsgemäßen Mittels als kosmetische Formulierung dar. Dabei kann es sich um jede beliebige kosmetische Formulierung handeln, die zur Pflege, Reinigung oder Verschönerung des Körpers dienlich ist, von Antihydrotika über Haarpflege bis hin zur Zahnpflege, um hier nur drei beliebig herausgegriffene Beispiele zu nennen.

Eine weitere bevorzugte Ausführungsform betrifft die Verwendung erfindungsgemäßer Mittel als Textilbehandlungsmittel. Dabei handelt es sich insbesondere um Waschmittel oder Textilnachbehandlungsmittel oder Nachspülmittel oder Weichspüler.

In einer weiteren bevorzugten Ausführungsform handelt es sich um die Verwendung eines erfindungsgemäßen Mittels zur Reinigung und/oder Pflege harter Oberflächen, beispielsweise zur Reinigung von Geschirr, von Porzellan oder Keramik, von Böden oder Glas usw.

Eine weitere bevorzugte Ausführungsform besteht in der Verwendung eines erfindungsgemäßen Mittels zur Konditionierung keratiner Fasern, insbesondere unter Gebrauch eines Sprühspenders.

Eine andere bevorzugte Ausführungsform besteht in einem Erzeugnis, enthaltend ein erfindungsgemäßes Mittel und einen Sprühspender.

Bevorzugt ist der Sprühspender ein manuell aktivierbarer Sprühspender, insbesondere ausgewählt aus der Gruppe umfassend Aerosolsprühspender, selbst Druck aufbauende Sprühspender, Pumpsprühspender und Triggersprühspender, insbesondere Pumpsprühspender und Triggersprühspender, vorteilhafterweise mit einem Behälter aus transparentem Polyethylen oder Polyethylenterephthalat.

Solche und ähnliche Sprühspender oder damit verwandte Applikationsvorrichtungen sind handelsüblich, und sämtliche handelsüblichen Sprühspender oder verwandte Applikationsvorrichtungen kommen zur erfindungsgemäßen Applikation in Betracht.

Demgemäß ist ein weiterer Gegenstand der Erfindung ein Verfahren zur Behandlung harter oder weicher Substrate bzw. Substratoberflächen, bei dem eine wirksame Menge eines erfindungsgemäßen Mittels, vorzugsweise unter Verwendung eines eben beschriebenen Erzeugnisses, auf das zu behandelnde Substrat vorzugsweise durch Sprühen aufgebracht wird, mit der Maßgabe, daß das Mittel in flüssiger Form, insbesondere emulgiert vorliegt. Unter einer wirksamen Menge wird dabei ein Menge verstanden, die eine wunschgemäßes Behandlungsresultat ermöglicht. Diese Menge ist eine individuelle, die von vielen Faktoren wie z. B. Substrattyp und -zustand, gewünschtes bzw. zu erzielendes Resultat abhängt.

In einer bevorzugten Ausführungsform des eben genannten Verfahrens wird das erfindungsgemäße Mittel, insbesondere unter Verwendung eines erfindungsgemäßen Erzeugnisses, auf das zu behandelnde Substrat, insbesondere aus einer Entfernung von 10 bis 100 cm, vorzugsweise 20 bis 50 cm, besonders bevorzugt 25 bis 40 cm, äußerst bevorzugt etwa 30 cm, gesprüht.

Der besondere Vorteil der vorgenannten Gegenstände, die sich auf die Verwendung eines Sprühspenders beziehen, liegt darin, daß das versprühte Mittel besonders gut auf dem Substrat haftet und so eine effiziente Wirkstofffreisetzung ermöglicht.

Ein weiterer Gegenstand der Erfindung ist ein Konditioniersubstrat, welches mit einem erfindungsgemäßen Mittel beschichtet und/oder getränkt ist. Die Ausgestaltungsform des Imprägnier- bzw. Beschichtungsmittels bzw. Tränkmittels ist der vorstehenden Beschreibung zu entnehmen.

Konditioniersubstrate finden ihren Einsatz vor allem in der Textilbehandlung und insbesondere in Textiltrocknungsverfahren. Das Substratmaterial besteht vorzugsweise aus porösen flächigen Tüchern. Sie können aus einem faserigen oder zellulären flexiblen Material bestehen, das ausreichend thermische Stabilität zur Verwendung im Trockner aufweist und das ausreichende Mengen eines Imprägnierungs- bzw. Beschichtungsmittels zurückhalten kann, um Stoffe effektiv zu konditionieren, ohne dass während der Lagerung ein nennenswertes Auslaufen oder Ausbluten des Mittels erfolgt. Zu diesen Tüchern gehören Tücher aus gewebtem und ungewebtem synthetischen und natürlichen Fasern, Filz, Papier oder Schaumstoff, wie hydrophilem Polyurethanschaum.

Vorzugsweise werden hier herkömmliche Tücher aus ungewebtem Material (Vliese, insbesondere Viskosevliese) verwendet. Vliese sind im allgemeinen als adhesiv gebondete faserige Produkte definiert, die eine Matte oder geschichtete Faserstruktur aufweisen, oder solche, die Fasermatten umfassen, bei denen die Fasern zufällig oder in statistischer Anordnung verteilt sind. Die Fasern können natürlich sein, wie Wolle, Seide, Jute, Hanf, Baumwolle, Lein, Sisal oder Ramie; oder synthetisch, wie Rayon, Celluloseester, Polyvinylderivate, Polyolefine, Polyamide oder Polyester. Im allgemeinen ist jeder Faserdurchmesser bzw. -titer für die vorliegende Erfindung geeignet. Die hier eingesetzten ungewebten Stoffe neigen aufgrund der zufälligen oder statistischen Anordnung von Fasern in dem ungewebten Material, die ausgezeichnete Festigkeit in allen Richtungen verleihen, nicht zum Zerreißen oder Zerfallen, wenn sie zum Beispiel in einem haushaltsüblichen Wäschetrockner eingesetzt werden. Beispiele für ungewebte Stoffe, die sich als Substrate in der vorliegenden Erfindung eignen, sind beispielsweise aus WO 93/23603 bekannt. Bevorzugte poröse und flächige Konditionierungstücher bestehen aus einem oder verschiedenen Fasermaterialien, insbesondere aus Baumwolle, veredelter Baumwolle, Polyamid, Polyester oder Mischungen aus diesen. Vorzugsweise weisen die Konditioniersubstrate in Tuchform eine Fläche von 0,2 bis 0,005 m², vorzugsweise von 0,15 bis 0,01 m², insbesondere von 0,1 bis 0,03 m² und besonders bevorzugt von 0,09 bis 0,06 m² auf. Die Grammatur des Materials beträgt dabei üblicherweise zwischen 20 und 1000 g/m², vorzugsweise von 30 bis 500 g/m² und insbesondere von 50 bis 150 g/m². Konditioniersubstrate können durch Tränken oder Imprägnierung oder auch durch Aufschmelzen der erfindungsgemäßen Mittel oder Konditioniermittel auf ein Substrat erhalten werden.

Demgemäß ist ein weiterer Gegenstand dieser Erfindung ein Textilkonditionierverfahren bei dem ein oder mehrere Konditioniersubstrate gemäß der eben gemachten Ausführungen in einem Textiltrocknungsprozeß eingesetzt werden.

Ein weiterer Gegenstand dieser Erfindung ist die Verwendung einer auf harte und/oder weiche Substratoberflächen aufziehenden, zumindest eine kationische Ladung tragenden Verbindung zur Fixierung eines Oligomers, Polymers oder Copolymers, welche das Strukturelement gemäß Formel (1) wenigstens einmal enthält, auf harten und/oder weichen Substratoberflächen

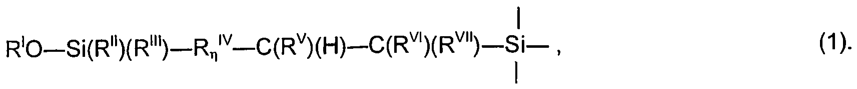

### Beispiele

Zur Beurteilung der erfindungsgemäßen Mittel wurden mehrere Vergleichsversuche in Form von Waschversuchen und Spülversuchen durchgeführt. Dabei wurden jeweils Waschmittel bzw. Weichspüler verglichen die, absolut betrachtet, nahezu dieselbe Konzentration an Duftstoff, hier Phenylethylalkohol, aufweisen. Der einzige Unterschied lag darin, daß im erfindungsgemäßen Fall der Duftstoff in Form eines Silikonderivates eingearbeitet wurde, während bei den Vergleichsversuchen der Duftstoff direkt eingearbeitet wurde.

An den Vergleichstests haben jeweils 8 Probanden teilgenommen, welche die Intensität des Duftes beurteilt haben, jeweils bezogen auf den Geruch des Produktes als solches, sowie den Geruch der Wäsche im feuchten wie in trockenem Zustand. Mit feuchtem Zustand ist gemeint, daß die feuchte Wäsche nach dem Schleudern aus der Trommel genommen wurde und ihr Duft beurteilt wurde. Die Wäsche wurde danach auf der Leine getrocknet. Der Duft der trockenen Wäsche wurde nach einem Tag, sowie nach 3, 7 und 14 Tagen beurteilt, wobei die trockene Wäsche sorgfältig voneinander getrennt in offenen Plastikbeuteln gelagert wurde. Dabei wurden die Muster in einem Paarvergleich beurteilt, wobei der Sieger des Vergleiches 1 Punkt und der Verlierer 0 Punkte erhält. D. h., daß die höchste Punktzahl auch als bestes Muster abgeschnitten hat, d. h. aber auch, daß die Muster, die mit 0 Punkten abgeschnitten haben, nicht geruchlos sind, sondern nur in dem Vergleich schlechter beurteilt wurden. Dabei wurde jeder Paarvergleich von jedem Probanden für jedes Paar 4 mal wiederholt.

In den erfindungsgemäßen Beispielen gelangten die Silikonderivate A und B zur Anwendung. Es handelt sich dabei um die nachfolgenden Verbindungen:

### Silikonderivat A:

Ph(CH₂)₂O-SiMe₂-(CH₂)₂-SiMe₂-O-SiMe₂-(CH₂)₂-SiMe₂-O(CH₂)₂Ph

Der Anteil des Phenylethylalkoholrestes beträgt ca. 45 Gew.-%, bezogen auf das Silikonderivat A.

### Silikonderivat B:

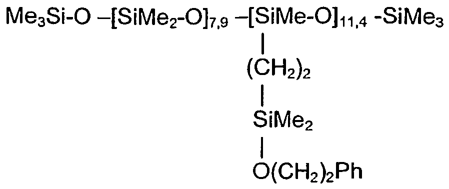

Der Anteil des Phenylethylalkoholrest beträgt ca. 37 Gew.-%, bezogen auf das Silikonderivat B

### Beispiel 1:

Grundmasse: 99 Gew.-Teile eines handelsüblichen parfumfreien Waschmittels und 1 Gewichtsteil Rewoquat® WE 18; (Di-(Talgcarboxyethyl)-hydroxyethyl-methylammonium-methosulfat; Hersteller: Degussa) und die in Tabelle 1 angegebene Menge Parfumöl bzw. Silikonderivat; Gesamtmenge Waschmittel inclusive Rewoquat® WE 18: 146 g; Waschtemperatur: 60°C; Standardwaschprogramm Buntwäsche; Waschmaschine Typ Miele Novotronic W135

**Tabelle 1**

| Präferenz der Intensität des Dufteindruckes | | | | | | |
|---|---|---|---|---|---|---|
| Parfumöl | Produkt | Wäsche feucht | Wäsche trocken 1 Tag | Wäsche trocken 3 Tage | Wäsche trocken 7 Tage | Wäsche trocken 14 Tage |
| 0,4 Gew.-Teile Phenylethylalkohol | 8 | 1 | 0 | 1 | 0 | 0 |
| 0,89 Gew.-Teile Silikonderivat **A** | 0 | 4 | 7 | 6 | 8 | 8 |

Bei der feuchten Wäsche konnten 3 Probanden keinen Unterschied der Intensität des Dufteindruckes wahrnehmen, bei der 1 Tag und bei der 3 Tage trockenen Wäsche konnte jeweils 1 Proband keinen Unterschied der Intensität des Dufteindruckes wahrnehmen.

### Beispiel 2:

Grundmasse: 99 Gew.-Teile eines handelsüblichen parfumfreien Waschmittels und 1 Gewichtsteil Rewoquat® WE 18; (Di-(Talgcarboxyethyl)-hydroxyethyl-methylammonium-methosulfat; Hersteller: Degussa) und die in Tabelle 2 angegebene Menge Parfumöl bzw. Silikonderivat; Gesamtmenge Waschmittel inclusive Rewoquat® WE 18: 146 g; Waschtemperatur: 60°C; Standardwaschprogramm Buntwäsche; Waschmaschine Typ Miele Novotronic W135

**Tabelle 2**

| Präferenz der Intensität des Dufteindruckes | | | | | | |
|---|---|---|---|---|---|---|
| Parfumöl | Produkt | Wäsche feucht | Wäsche trocken 1 Tag | Wäsche trocken 3 Tage | Wäsche trocken 7 Tage | Wäsche trocken 14 Tage |
| 0,4 Gew.-Teile Phenylethylalkohol | 8 | 7 | 0 | 0 | 0 | 0 |
| 1,08 Gew.-Teile Silikonderivat **B** | 0 | 0 | 5 | 7 | 6 | 8 |

Bei der feuchten Wäsche konnte 1 Proband keinen Unterschied der Intensität des Dufteindruckes wahrnehmen, bei der 1 Tag trockenen Wäsche konnten 3 Probanden keinen Unterschied wahrnehmen, bei der 3 Tage trockenen Wäsche konnte 1 Proband keinen Unterschied wahrnehmen, bei der 7 Tage trockenen Wäsche konnten 2 Probanden keinen Unterschied wahrnehmen.

### Beispiel 3:

Grundmasse: 99 Gew.-Teile eines handelsüblichen parfumfreien Weichspülers enthaltend ca. 17 Gew.-Teile Rewoquat® WE 18 (Di-(Talgcarboxyethyl)-hydroxyethyl-methylammonium-methosulfat) und die in Tabelle 3 angegebene Menge Parfumöl bzw. Silikonderivat; Gesamtmenge Weichspüler: 36 g; Waschmaschine Typ Miele Novotronic W135; Standardspülgang bei Spültemperatur: 20°C ohne vorangegangenen Waschgang

**Tabelle 3**

| Präferenz der Intensität des Dufteindruckes | | | | | | |
|---|---|---|---|---|---|---|
| Parfumöl | Produkt | Wäsche feucht | Wäsche trocken 1 Tag | Wäsche trocken 3 Tage | Wäsche trocken 7 Tage | Wäsche trocken 14 Tage |
| 0,9 Gew.-Teile Phenylethylalkohol | 8 | 7 | 0 | 0 | 0 | 0 |
| 2,0 Gew.-Teile Silikonderivat **A** | 0 | 0 | 8 | 8 | 8 | 8 |

Bei der feuchten Wäsche konnte 1 Probanden keinen Unterschied der Intensität des Dufteindruckes wahrnehmen.

### Beispiel 4:

Grundmasse: 99 Gew.-Teile eines handelsüblichen parfumfreien Weichspülers enthaltend ca. 17 Gew.-Teile Rewoquat® WE 18 (Di-(Talgcarboxyethyl)-hydroxyethyl-methylammonium-methosulfat) und die in Tabelle 4 angegebene Menge Parfumöl bzw. Silikonderivat; Gesamtmenge Weichspüler: 36 g; Waschmaschine Typ Miele Novotronic W135; Standardspülgang bei Spültemperatur: 20°C ohne vorangegangenen Waschgang

**Tabelle 4**

| Präferenz der Intensität des Dufteindruckes | | | | | | |
|---|---|---|---|---|---|---|
| Parfumöl | Produkt | Wäsche feucht | Wäsche trocken 1 Tag | Wäsche trocken 3 Tage | Wäsche trocken 7 Tage | Wäsche trocken 14 Tage |
| 0,9 Gew.-Teile Phenylethylalkohol | 7 | 8 | 2 | 0 | 0 | 0 |
| 2,43 Gew.-Teile Silikonderivat **B** | 1 | 0 | 5 | 7 | 6 | 8 |

Bei der 1 Tag und ebenso bei der 3 Tage trockenen Wäsche konnte jeweils 1 Proband keinen Unterschied der Intensität des Dufteindruckes wahrnehmen, bei der 7 Tage trockenen Wäsche konnten 2 Probanden keinen Unterschied wahrnehmen.

Wie die vorgelegten Versuche zeigen, duftet in allen Beispielen das Produkt als solches sowie die feuchte Wäsche intensiver, wenn das Produkt mit dem bloßen Phenylethylalkohol versetzt ist. Das ist zu erwarten, da dem bloßen Phenylethylalkohol keine Retard-Wirkung innewohnt. Wenn dagegen die trockene Wäsche betrachtet wird, duftet die Wäsche dagegen intensiver, insbesondere nach langer Zeit, d. h. nach 7 oder 14 Tagen, wenn in das Mittel das entsprechende Silikonderivat eingearbeitet wurde, und nicht der bloße Phenylethylalkohol. Hier zeigt sich die Retard-Wirkung der Silikonderivate, die eine kontinuierliche, zeitverzögerte Duftfreisetzung bewirken und so für ein lange anhaltendes Dufterlebnis sorgen.

Das belegt, daß die Silikonderivate in der Lage sind, sehr gut auf die behandelten Textilien aufzuziehen und dort eine langanhaltende Duftwirkung zu entfalten, da sie retardiert freigesetzt werden.

## Patentansprüche

1. Mittel, insbesondere Wasch- oder Reinigungsmittel oder Konditioniermittel oder kosmetisches Mittel, umfassend wenigstens ein Oligomer, Polymer oder Copolymer, welches ein Strukturelement gemäß Formel (1) wenigstens einmal enthält, 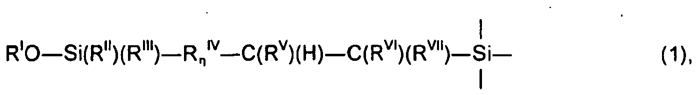 wobei R^{II}, R^{III} unabhängig voneinander jeweils für einen aliphatischen oder aromatischen, geradkettigen oder verzweigten, gesättigten oder ungesättigten, substituierten oder unsubstituierten Kohlenwasserstoffrest steht, der jeweils Heteroatome wie Sauerstoff, Stickstoff, Schwefel oder Halogene oder andere enthalten kann, und wobei R_{η}^{IV} für ein Kohlenstoff-Brückenglied steht, welches ein aliphatischer oder aromatischer, geradkettiger oder verzweigter, gesättigter oder ungesättigter, substituierter oder unsubstituierter Kohlenwasserstoffrest ist, der jeweils Heteroatome wie Sauerstoff, Stickstoff, Schwefel oder Halogene oder andere enthalten kann, wobei die Laufzahl η 0 bis 10 beträgt, und wobei R^{V}, R^{VI}, R^{VII} unabhängig voneinander jeweils für Wasserstoff oder einen aliphatischen oder aromatischen, geradkettigen oder verzweigten, gesättigten oder ungesättigten, substituierten oder unsubstituierten Kohlenwasserstoffrest stehen, der jeweils Heteroatome wie Sauerstoff, Stickstoff, Schwefel oder Halogene oder andere enthalten kann, und wobei das in der Formel 1 endständige Silicium an seinen verbliebenen drei Valenzen unabhängig voneinander beliebige Reste des Oligomers, Polymers oder Copolymers aufweist, und wobei R'O entweder einen Rest darstellt, der eine Duftstoff-Alkoxy-Gruppe und/oder Biozid-Alkoxy-Gruppe ist, die abgeleitet ist von dem korrespondierenden Duftstoff- und/oder Biozid-Alkohol R^{I}OH, oder wobei R'O einen Rest darstellt, der von einem enolisierbaren Duftstoff- und/oder Biozid- Ester, Keton oder Aldehyd abgeleitet ist,
und wobei das Mittel zusätzlich mindestens eine auf harte und/oder weiche Substratober-flächen aufziehende Verbindung enthält, welche zumindest eine kationische Ladung trägt.

2. Mittel nach Anspruch 1, **dadurch gekennzeichnet, dass** es ein duftgebendes, biozides und/oder duftgebendes biozides Mittel ist.

3. Mittel nach einem der Ansprüche 1-2, enthaltend wenigstens ein Siliconoligomer, -polymer oder -copolymer, das bei der Hydrolyse einen duftenden und/oder bioziden Alkohol, Aldehyd, Keton oder Ester freisetzt, welcher vorzugsweise durch Umsetzung mit einem olefinischen Silan eingeführt worden ist.

4. Mittel nach Anspruch 3, **dadurch gekennzeichnet, dass** das olefinische Silan ein Reaktionsprodukt eines duftenden und/oder bioziden Alkohols, Aldehyds, Ketons oder Esters und einem olefinischen Halosilan oder olefinischen Silikonalkoxid ist.

5. Mittel nach einem der Ansprüche 3-4, **dadurch gekennzeichnet, dass** es sich um ein olefinisches Silan gemäß Formel (2) handelt
(R^{VIII}O)ₐ(R^{IX}O)_{b}(R^{x}O)_{c}(R^{XI})_{d} (R^{XII})ₑSiR^{XIII} (2)
wobei R^{VIII}O, R^{IX}O und R^{X}O jeweils unabhängig voneinander Duftstoff-Alkoxy-Gruppen darstellen, die abgeleitet sind von den korrespondierenden Duftstoff- Alkoholen R^{VIII}OH, R^{IX}OH und R^{X}OH,
wobei R^{XI}, R^{XII} ausgewählt sind aus der Gruppe monovalente C₁₋₄₀ Kohlenwasserstoffreste und monovalente C₁₋₄₀ Alkoxy-Reste, R^{XIII} ein C₂₋₄₀ monovalenter ungesättigter Kohlenwasserstoffrest mit olefinischer Endgruppe ist, und a einen Wert von 1 - 3 hat, b, c, d, e Werte von 0-2 haben, mit der Maßgabe, dass a+b+c+d+e=3 und a, b, c, d, e ganze Zahlen sind.

6. Mittel nach einem der Ansprüche 3- 4, **dadurch gekennzeichnet, dass** es sich um ein olefinisches Silan gemäß Formel (3) handelt
(R^{XIV})ₐ(R^{XV})_{b}(R^{XVI})_{c}(R^{XI})_{d} (R^{XII})ₑSiR^{XIII} (3)
wobei R^{XIV}, R^{XV} und R^{XVI} jeweils unabhängig voneinander die Formel (4) R^{XVII}(R^{XVIII})C=C(O-)R^{XIX} (4) aufweisen, R^{XVII}, R^{XVIII} und R^{XIX} unabhängig voneinander für jedes R^{XIV}, R^{XV} und R^{XVI} gewählt sind, R^{XI}, R^{XII} ausgewählt sind aus der Gruppe monovalente C₁₋₄₀ Kohlenwasserstoffreste und monovalente C₁₋₄₀ Alkoxy-Reste, R^{XIII} ein C₂₋₄₀ monovalenter ungesättigter Kohlenwasserstoffrest mit olefinischer Endgruppe ist, und a einen Wert von 1-3 hat, b, c, d, e Werte von 0-2 haben, mit der Maßgabe, dass a+b+c+d+e=3 und a, b, c, d, e ganze Zahlen sind, und R^{XVII}, R^{XVIII} und R^{XIX} ausgewählt sind aus der Gruppe bestehend aus Wasserstoff und monovalente C₁₋₁₀₀ Kohlenwasserstoffreste.

7. Mittel nach Anspruch 6, wobei R^{XIV}, R^{XV} und R^{XVI} jeweils unabhängig voneinander die Formel (4) R^{XVII}(R^{XVIII})C=C(O-)R^{XIX} (4) aufweisen und abgeleitet sind aus der Gruppe der nachfolgenden Aldehyde, Ketone oder Ester ausgewählt aus 3-Methyl-3(3-(1- Methylethylphenyl))propanal), 2-Methyl-3-(4-t-butylphenyl)propanal, 3-Phenylpropional, 2-Phenylpropional, Propional, Isobutyral, 2-Methylbutyral, Hexanal, Octanal, Nonanal, Decanal, 3,7-Dimethyl-1-al, p- Tolylacetaldehyd, Phenylacetaldehyd, 4-(3)(4-Methyl-3-pentenyl)-3- cyclohexen-carbaldehyd, 2,6-Dimethyl-5-heptenal, 3,7-Dimethyl-2,6-octadienal, trans-4-Decenal, Cyclamenaldehyd, 4-(p-Methoxyphenyl)- 2butanon, Acetophenon, 2-Pentanon, 2-Butanon, 2-Heptanon, 3- Heptanon, 2-Decanon, 3-Penten-2-on, 6-Methyl-5-hepten-2-on, Geranylaceton, 5-Methyl-alpha-ionon, 2-Acetonaphton, 2-Methyl-3-phenylpropan-2-ylacetat, Linalylacetat, Menthanylacetat, 2-Phenylethyl-acetat, Tetrahydrolinalylacetat, Phenethylpropionat, Phenethylhexanoat, Butyl-acetat, Phenoxyethylisobutyrat, p-tert. -Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat, lineare Alkanale mit 8-18 C-Atomen. Citral, Citronellal, Citronellyloxy-acetaldehyd, Cyclamenaldehyd, Lilial, Bourgeonal, die Jonone, α-Isomethylionon und Methylcedrylketon.

8. Mittel nach Anspruch 7, **dadurch gekennzeichnet, dass** die Duftstoff- und/oder Biozid-Alkoxy-Gruppen R^{VIII}O, R^{IX}O und R^{X}O jeweils abgeleitet sind von Duftstoff- und/oder Biozidalkoholen, ausgewählt aus der Gruppe 2-Methylbutanol, 3-Pentanol, n-Pentanol, 2-Pentanol, n-Hexanol, 2-Methylpentanol, 1-Decanol, Sandela, Nonadol, Dimetol, Thymol, 1-Heptanol, Menthol, Eugenol, Vanillin, o-Vanillin, 4-(p-Hydroxyphenyl)-2-Butanon, Syringealdehyd, Prenol, cis-3-Hexanol, trans- 3-Hexanol, cis4-Heptenol, trans-2-Octenol, trans-2-cis-6-Nonadienol, Nerol, Ebanol, Crotylalkohol, Oleylalkohol, Linalool, α-Terpineol, β-Phenethylalkohol, Zimtalkohol, Benzylalkohol, α -Methylbenzylalkohol, Nonylalkohol, 1-Octanol, 3-Octanol, Phenethylsalicylat, Hydrozimtalkohol, cis-6-Nonen-1-ol, trans-2-Nonen-1-ol, Methylsalicylat, cis-3-Octen-ol, Anisylalkohol, Carvacrol, Dihydrocarveol, Benzylsalicylat, Tetrahydrogeraniol, Ethylsalicylat, Ethylvanillin, Isoeugenol, Isopulegol, Laurylalkohol, Tetrahydrolinalool, 2-Phenoxyethanol, Citronellol, Farnesol und Geraniol.

9. Mittel, nach einem der Ansprüche 1-8, **dadurch gekennzeichnet, dass** die Molmasse des Oligomers, Polymers oder Copolymers bis etwa 300000 beträgt, vorzugsweise bis 100000, besonders bevorzugt im Bereich von etwa 150 bis etwa 30000 liegt.

10. Mittel, nach einem der Ansprüche 1-9, **dadurch gekennzeichnet, dass** der Anteil des Duftstoffrestes bzw. bioziden Restes an der Gesamtmasse des Oligomers, Polymers oder Copolymers bis zu 80 Gew.-%, vorzugsweise bis zu 70 Gew.-% beträgt, insbesondere zwischen 0,001 und 60 Gew.-% liegt, jeweils bezogen auf das gesamte Mittel.

11. Mittel, nach einem der Ansprüche 1-10, **dadurch gekennzeichnet, dass** das Oligomer, Polymer oder Copolymer im wesentlichen unverzweigt ist, vorzugsweise zu mindestens 50%, vorteilhafterweise zu mindestens 60 %, insbesondere zu mindestens 70% linear ist.

12. Mittel, nach einem der Ansprüche 1-11, **dadurch gekennzeichnet, dass** das Oligomer, Polymer oder Copolymer der Formel (5) gehorcht:
M_{f}M^{F}_{g}DₕD^{F}ᵢTⱼT^{F}ₖQₗ (5)
mit M: R^{XX}R^{XXI}R^{XXII}SiO_{1/2}; M^{F}: R^{XX}R^{XXI}R^{F}SiO_{1/2}; D: R^{XXIII}R^{XXIV}SiO_{2/2}; D^{F}: R^{XXIII}R^{F}SiO_{2/2}; T: R^{XXV}SiO_{3/2}; T^{F}: R^{F}SiO_{3/2} Q: SiO_{4/2}, wobei
R^{XX}, R^{XXI}, R^{XXII}, R^{XXIII}, R^{XXIV}, R^{XXV} jeweils unabhängig voneinander ausgewählt sind für jedes M, M^{F}, D, D^{F}, T und T^{F} aus der Gruppe der C₁₋₄₀ monovalenten, geradkettigen oder verzweigten, gesättigten oder ungesättigten Alkyl- oder Alkoxyreste oder C₁₋₄₀ monovalente Aryl- oder Aryloxyreste, wobei die vorgenannten Alkyl-, Alkoxy-, Aryl-, Aryloxyreste substituiert sein können, sowie Heteroatome wie Sauerstoff, Stickstoff, Schwefel oder Halogene oder andere enthalten können, wobei f , g positive Zahlen sind, h, i, j, k, I sind positive Zahlen oder gleich null, wobei wenigstens einer der h, i, j, k, I ungleich null ist und zumindest einer der g, i, oder k gleich 1 ist oder größer als 1, und
wobei R^{F} abgeleitet ist von einem der vorgenannten Reste (R^{VIII}O)ₐ(R^{IX}O)_{b}(R^{X}O)_{c}(R^{XI})_{d}(R^{XII})_{d}(RII)ₑSiR^{XIII} (gemäß Formel (2)) und/oder (R^{XIV})ₐ(R^{XV})_{b}(R^{XVI})_{c}(R^{XVI})_{d}(R^{XII})ₑSiR^{XIII} (gemäß Formel (3)), wobei dieser Rest R^{F} über ein bivalentes C₂₋₄₀ Kohlenwasserstoffbrückenglied, das abgeleitet ist von R^{XIII} (ein C₂₋₄₀ monovalenter ungesättigter Kohlenwasserstoffrest mit olefinischer Endgruppe) mit einem Si-Atom des Oligomers, Polymers oder Copolymers verbunden ist.

13. Mittel, nach einem der Ansprüche 1-12, **dadurch gekennzeichnet, dass** das Oligomer, Polymer oder Copolymer ausgewählt ist aus folgenden Formeln: 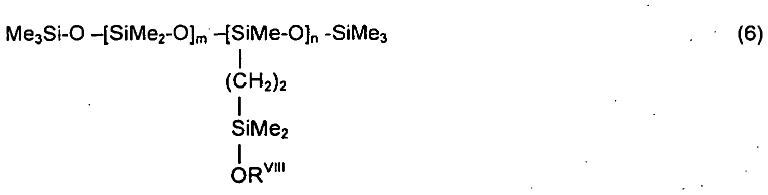 wobei OR^{VIII} für einen Duftstoff- oder Biozid-Alkoxy-Rest steht, insbesondere für einen Phenylethylalkohol-Rest, wobei m und n jeweils einen positiven Wert haben, mit der Maßgabe dass das resultierende Silikon eine Molmasse von zumindest 150 erreicht
und/oder
R^{VIII}O-SiMe₂-(CH₂)₂-[SiMe₂-O]ₚ-SiMe₂-(CH₂)₂-SiMe₂-OR^{VIII} (7)
wobei OR^{VIII} für einen Duftstoff- oder Biozid-Alkoxy-Rest steht, insbesondere für eine Phenylethylalkohol-Rest, mit p größer 0, mit der Maßgabe dass das resultierende Silikon eine Molmasse von zumindest 150 erreicht.

14. Mittel nach einem der Ansprüche 1-13, **dadurch gekennzeichnet, dass** das Oligomer, Polymer oder Copolymer in Mengen größer 0,001 Gew.-%, vorteilhafterweise von 0,002 bis 10 Gew.-%, insbesondere von 0,01 bis 5 Gew.-%, besonders bevorzugt von 0,02 bis 3 Gew.-% und ganz besonders bevorzugt in Mengen von 0,05 bis 2 Gew.-% enthalten ist, jeweils bezogen auf das gesamte Mittel.

15. Mittel nach einem der Ansprüche 1-14, **dadurch gekennzeichnet, dass** die auf harte und/oder weiche Substratoberflächen aufziehende, zumindest eine kationische Ladung tragende Verbindung in Mengen größer 0.01 Gew.-%, insbesondere von 0,02 bis 45 Gew.-%, vorteilhafterweise von 5 bis 40 Gew.-%, in besonders vorteilhafter Weise von 10-35 Gew.-%, jeweils bezogen auf das gesamte Mittel, enthalten ist.

16. Mittel nach einem der Ansprüche 1-15, **dadurch gekennzeichnet, dass** es sich bei der auf harte und/oder weiche Substratoberflächen aufziehenden, zumindest eine kationische Ladung tragenden Verbindung um eine Verbindung handelt, die ausgewählt ist aus der Gruppe der kationischen oder amphoteren Emulgatoren, kationischen Tenside, zwitterionischen Verbindungen, Ampholyte, Amphotenside, Betaine und/oder kationische oder amphotere Polymere.

17. Mittel nach einem der Ansprüche 1-16, **dadurch gekennzeichnet, dass** es sich bei der auf harte und/oder weiche Substratoberflächen aufziehenden Verbindung um eine Verbindung handelt, die in wässrigem Medium bei pH-Werten kleiner als 4, vorzugsweise kleiner als 5, vorteilhafterweise kleiner als 6, in besonders vorteilhafter Weise kleiner 7, in ganz besonders vorteilhafter Weise kleiner als 8, in überaus vorteilhafter Weise kleiner 9, insbesondere kleiner als 10, wenigstens eine kationische Ladung aufweist, wobei der pH-Wert bei 20°C gemessen wird.

18. Mittel nach einem der Ansprüche 1-17, **dadurch gekennzeichnet, dass** es sich bei der auf harte und/oder weiche Substratoberflächen aufziehenden Verbindung um quartäre Ammoniumverbindungen handelt, vorzugsweise um alkylierte quartäre Ammoniumverbindungen, von denen mindestens eine Alkylkette durch eine Estergruppe und/oder Amidogruppe unterbrochen ist.

19. Mittel nach einem der Ansprüche 1-18, **dadurch gekennzeichnet, dass** es sich bei der auf harte und/oder weiche Substratoberflächen aufziehenden Verbindung um eine quartäre Ammoniumverbindung, ausgewählt aus den nachfolgenden Formeln (9): 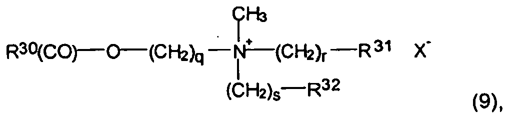 hierbei steht R³⁰ für einen aliphatischen Alkylrest mit 12 bis 22 Kohlenstoffatomen mit 0, 1, 2 oder 3 Doppelbindungen; R³¹ steht für H, OH oder insbesondere O(CO)R^{a}, R³² steht unabhängig von R³¹ für H, OH oder O(CO)R^{b}, wobei R^{a} und R^{b} unabhängig voneinander jeweils für einen aliphatischen Alkylrest mit 12 bis 22 Kohlenstoffatomen mit 0, 1, 2 oder 3 Doppelbindungen steht, q, r und s können jeweils unabhängig voneinander den Wert 1, 2 oder 3 haben, X- ist ein passendes Anion, vorzugsweise ein Halogenid-, Methosulfat-, Methophosphat- oder Phosphation sowie Mischungen aus diesen sein,
und/oder der Formel (12) handelt: 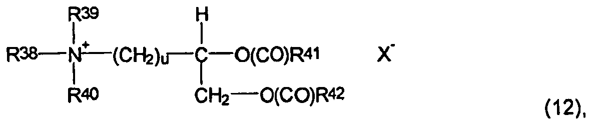 R³⁸ , R³⁹ und R⁴⁰ unabhängig voneinander für eine C₁₋₄-Alkyl-, Alkenyl- oder Hydroxyalkylgruppe steht, R⁴¹ und R⁴² jeweils unabhängig ausgewählt eine C₈₋₂₈-Alkylgruppe mit 0, 1, 2 oder 3 Doppelbindungen darstellt und u eine Zahl zwischen 0 und 5 ist, X⁻ ist ein passendes Anion, vorzugsweise ein Halogenid-, Methosulfat-, Methophosphat- oder Phosphation sowie Mischungen aus diesen sein.

20. Mittel nach einem der Ansprüche 1-19, **dadurch gekennzeichnet, dass** es sich bei der auf harte und/oder weiche Substratoberftachen aufziehenden Verbindung um N-Methyl-N(2-hydroxyethyl)-N,N-(ditalgacyloxyethyl)ammonium-methosulfat oder um N-Methyl-N(2-hydroxyethyl)-N,N-(dipalmitoylethyl)ammonium-methosulfat handelt.

21. Mittel nach einem der Ansprüche 1-20, **dadurch gekennzeichnet, dass** es sich bei der auf harte und/oder weiche Substratoberflächen aufziehenden, zumindest eine kationische Ladung tragenden Verbindung um eine zwitterionische Verbindung gemäß Formel (18) handelt 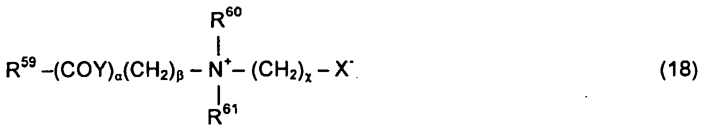 wobei das R⁵⁹ für eine C₆₋₂₈-Alkyl- oder Alkenylgruppe steht, und wobei R⁶⁰ und R⁶¹ jeweils, unabhängig voneinander, C₁₋₄ Alkyl-Gruppen sind, und wobei α für die Zahl 0 oder 1 steht, β und χ jeweils, unabhängig voneinander, aus ganzen Zahlen von 1 bis 4 ausgewählt sind, und wobei Y Sauerstoff oder Stickstoff ist, und wobei X ein kompatibles Anion ist.

22. Mittel nach einem der Ansprüche 1-21, **dadurch gekennzeichnet, dass** es sich bei der auf harte und/oder weiche Substratoberflächen aufziehenden, zumindest eine kationische Ladung tragenden Verbindung, um ein Alkylamidoalkylendimethylcarbonsäure-Betain gemäß Formel (19) handelt: 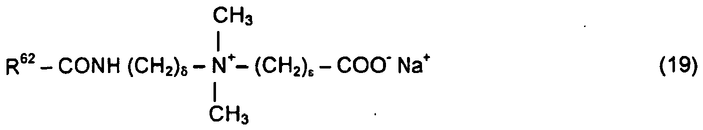 wobei δ und ε, unabhängig voneinander, ganze Zahlen von 1-4 sind, vorzugsweise ist δ gleich 2 oder 3 und ε gleich 2 oder 3 und wobei R⁶² für eine C₁₀₋₁₈-Alkylkette oder Mischungen davon steht.

23. Mittel nach einem der Ansprüche 1-22, **dadurch gekennzeichnet, dass** es sich bei der auf harte und/oder weiche Substratoberflächen aufziehenden, zumindest eine kationische Ladung tragenden Verbindung, um ein kationisches Nitril gemäß Formel (14) handelt 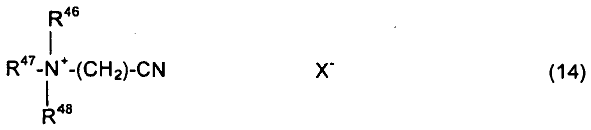 in der R⁴⁶ für -H, -CH₃, einen C₂₋₂₄-Alkyl- oder -Alkenylrest, einen substituierten C₂₋₂₄-Alkyl-oder -Alkenylrest mit mindestens einem Substituenten aus der Gruppe -Cl, -Br, -OH, -NH₂, -CN, einen Alkyl- oder Alkenylarylrest mit einer C₁₋₂₄-Alkylgruppe, oder für einen substituierten Alkyl- oder Alkenylarylrest mit einer C₁₋₂₄-Alkylgruppe und mindestens einem weiteren Substituenten am aromatischen Ring steht, R⁴⁷ und R⁴⁸ unabhängig voneinander ausgewählt sind aus -CH₂-CN, -CH₃, -CH₂-CH₃, -CH₂-CH₂-CH₃, -CH(CH₃)-CH₃, -CH₂-OH, -CH₂-CH₂-OH, -CH(OH)-CH₃, -CH₂-CH₂-CH₂-OH, -CH₂-CH(OH)-CH₃, -CH(OH)-CH₂-CH₃, -(CH₂CH₂-O)ₙH mit n gleich 1, 2, 3, 4, 5 oder 6 und X ein Anion ist.

24. Mittel nach einem der Ansprüche 1-23, **dadurch gekennzeichnet, dass** es ein kationisches Nitril gemäß Formel (15) beinhaltet 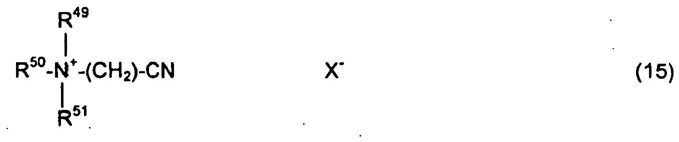 in der R⁴⁹, R⁵⁰ und R⁵¹ unabhängig voneinander ausgewählt sind aus -CH₃, -CH₂-CH₃, -CH₂-CH₂-CH₃, -CH(CH₃)-CH₃, wobei R⁴⁹ zusätzlich auch -H sein kann und X ein Anion ist, wobei vorzugsweise R⁵⁰ = R⁵¹ = -CH₃ und insbesondere R⁴⁹ = R⁵⁰ = R⁵¹ = -CH₃ gilt und Verbindungen der Formeln (CH₃)₃N⁽⁺⁾CH₂-CN X⁻, (CH₃CH₂)₃N⁽⁺⁾CH₂-CN X⁻, (CH₃CH₂CH₂)N⁽⁺⁾CH₂-CN X⁻, (CH₃CH(CH₃))₃N⁽⁺⁾CH₂-CN X⁻, oder (HO-CH₂-CH₂)₃N⁽⁺⁾CH₂-CN X⁻ besonders bevorzugt sind.

25. Mittel nach einem der Ansprüche 23-24, **dadurch gekennzeichnet, dass** X⁻ für ein Anion steht, das aus der Gruppe Chlorid, Bromid, Iodid, Hydrogensulfat, Methosulfat, Laurylsulfat, Dodecylbenzolsulfonat, p-Toluolsulfonat (Tosylat), Cumolsulfonat oder Xylolsulfonat oder deren Mischungen ausgewählt ist.

26. Mittel nach einem der Ansprüche 1-25, **dadurch gekennzeichnet, dass** das Mittel in fester, dispergierter, pulvriger, granulärer oder gepresster Form vorliegt, mit der Maßgabe, dass es, wenn es in gepresster Form vorliegt, insbesondere in Form einer Tabletten vorliegt, die aus einer einzigen oder mehreren Phasen besteht.

27. Mittel nach einem der vorigen Ansprüche 1-25, **dadurch gekennzeichnet, dass** das Mittel in gelförmiger oder flüssiger Form, insbesondere emulgiert vorliegt, wobei es vorzugsweise bis zu 95 Gew.-%, vorteilhafterweise 20 bis 90 Gew.-% und besonders bevorzugt 50-80 Gew.-% eines oder mehrerer Lösungsmittel enthält.

28. Mittel nach einem der Ansprüche 1-27, **dadurch gekennzeichnet, dass** es wenigstens eine weitere üblicherweise in Wasch- oder Reinigungsmitteln enthaltene Substanz, vorzugsweise eine Substanz aus der Gruppe der Tenside, Buildersubstanzen (anorganische und organische Buildersubstanzen), Bleichmittel, Bleichaktivatoren, Bleichstabilisatoren, Bleichkatalysatoren, Enzyme, spezielle Polymere (beispielsweise solche mit Cobuilder-Eigenschaften), Vergrauungsinhibitoren, optische Aufheller, UV-Schutzsubstanzen, Soil Repellents, Elektrolyte, Farbmittel, Riechstoffe, Duftstoffe, Parfumträger, pH-Stellmittel, Komplexbildner, Fluoreszenzmittel, Schauminhibitoren, Knitterschutzmittel, Antioxidantien, quartäre Ammoniumverbindungen, Antistatika, Bügelhilfsmittel, UV-Absorber, Antiredepositionsmittel, Germizide, antimikrobielle Wirkstoffe, Fungizide, Viskositätsregulatoren, Perlglanzgeber, Farbübertragungsinhibitoren, Einlaufverhinderer, Korrosionsinhibitoren, Konservierungsmittel, Weichmacher, Weichspüler, Proteinhydrolysate, Phobier- und Imprägniermittel, Hydrotrope, Silikonöle sowie Quell- und Schiebefestmittel enthält.

29. Verwendung eines Mittels nach einem der vorigen Ansprüche 1-28 als kosmetische Formulierung.

30. Verwendung eines Mittels nach einem der vorigen Ansprüche 1-28 als Textilbehandlungsmittel, insbesondere als Waschmittel oder Nachbehandlungsmittel oder Nachspülmittel oder als Weichspüler.

31. Verwendung eines Mittels nach einem der vorigen Ansprüche 1-28 zur Konditionierung keratiner Fasern.

32. Verfahren zur Behandlung von harten und/oder weichen Subtratoberflächen, **dadurch gekennzeichnet, dass** eine wirksame Menge eines Mittel nach einem der Ansprüche 1-28 unter Verwendung eines Sprühspenders auf das zu behandelnde Substrat aufgebracht wird, mit der Maßgabe, dass das Mittel in flüssiger Form, insbesondere emulgiert vorliegt.

33. Verwendung eines Mittels nach einem der vorigen Ansprüche 1-28 zur Reinigung und/oder Pflege harter Oberflächen.

34. Erzeugnis, enthaltend ein Mittel nach einem der Ansprüche 1-28 und einen Sprühspender.

35. Konditioniersubstrat **dadurch gekennzeichnet, dass** es ein mit einem Mittel nach einem der Ansprüche 1-28 getränktes und/oder beschichtetes Substrat ist.

36. Konditioniersubstrat nach Anspruch 35 **dadurch gekennzeichnet, dass** das Substrat aus einem Vliesmaterial, insbesondere einem Viskosevlies besteht.

37. Konditioniersubstrat nach einem der Ansprüche 35-36, **dadurch gekennzeichnet, dass** das Substrat eine Grammatur zwischen 20 bis 1000 g/m², insbesondere von 30 bis 500 g/m² aufweist.

38. Konditioniersubstrat nach einem der Ansprüche 35-37, **dadurch gekennzeichnet, dass** das Substrat eine Größe von 0,2 bis 0,005 m² aufweist.

39. Verfahren zur Textilkonditionierung, **dadurch gekennzeichnet, dass** ein oder mehrere Konditioniersubstrate nach einem der Ansprüche 35-38 in einem Textiltrocknungsprozeß eingesetzt werden.

40. Verwendung einer auf harte und/oder weiche Substratoberflächen aufziehenden, zumindest eine kationische Ladung tragenden Verbindung zur Fixierung eines Oligomers, Polymers oder Copolymers, das das nachfolgende Strukturelement gemäß Formel (1) wenigstens einmal enthält, auf harten und/oder weichen Substratoberflächen: 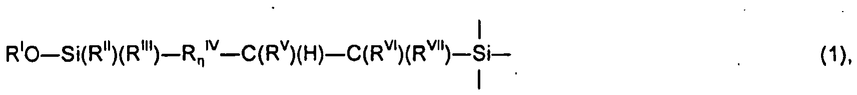 wobei R^{II}, R^{III} unabhängig voneinander jeweils für einen aliphatischen oder aromatischen, geradkettigen oder verzweigten, gesättigten oder ungesättigten, substituierten oder unsubstituierten Kohlenwasserstoffrest steht, der jeweils Heteroatome wie Sauerstoff, Stickstoff, Schwefel oder Halogene oder andere enthalten kann, und wobei R_{η}^{IV} für ein Kohlenstoff-Brückenglied steht, welches ein aliphatischer oder aromatischer, geradkettiger oder verzweigter, gesättigter oder ungesättigter, substituierter oder unsubstituierter Kohlenwasserstoffrest ist, der jeweils Heteroatome wie Sauerstoff, Stickstoff, Schwefel oder Halogene oder andere enthalten kann, wobei die Laufzahl η 0 bis 10 beträgt, und wobei R^{V}, R^{VI}, R^{VII} unabhängig voneinander jeweils für Wasserstoff oder einen aliphatischen oder aromatischen, geradkettigen oder verzweigten, gesättigten oder ungesättigten, substituierten oder unsubstituierten Kohlenwasserstoffrest stehen, der jeweils Heteroatome wie Sauerstoff, Stickstoff, Schwefel oder Halogene oder andere enthalten kann, und wobei das in der Formel 1 endständige Silicium an seinen verbliebenen drei Valenzen unabhängig voneinander beliebige Reste des Oligomers, Polymers oder Copolymers aufweist, und wobei R^{I}O entweder einen Rest dar stellt, der eine Duftstoff-Alkoxy-Gruppe und/oder Biozid-Alkoxy-Gruppe ist, die abgeleitet ist von dem korrespondierenden Duftstoff- und/oder Biozid-Alkohol R^{I}OH, oder R^{I}O stellt einen Rest dar, der abgeleitet ist von einem enolisierbaren Duftstoff- und/oder Biozid- Ester, Keton oder Aldehyd.

## Claims

1. Agents, particularly detergents or cleaning agents or conditioners or cosmetics, comprising at least one oligomer, polymer or copolymer that comprises a structural element in accordance with Formula (1) at least once, 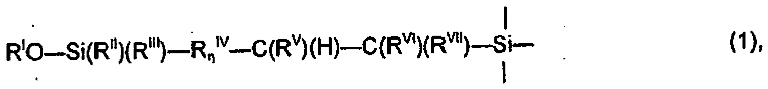 wherein R^{II}, R^{III}, independently of one another each stand for an aliphatic or aromatic, linear or branched, saturated or unsaturated, substituted or unsubstituted hydrocarbon group that, as appropriate, can comprise heteroatoms such as oxygen, nitrogen, sulfur or halogens or others and wherein R_{η}^{IV} stands for a carbon bridging member that is an aliphatic or aromatic, linear or branched, saturated or unsaturated, substituted or unsubstituted hydrocarbon group that respectively can comprise heteroatoms such as oxygen, nitrogen, sulfur or halogens or others, wherein the number η is 0 to 10, and wherein R^{V}, R^{VI}, R^{VII}, independently of each other each stand for hydrogen or an aliphatic or aromatic, linear or branched, saturated or unsaturated, substituted or unsubstituted hydrocarbon group that, as appropriate, can comprise heteroatoms such as oxygen, nitrogen, sulfur or halogens or others, and wherein the terminal silicon in Formula 1 has its remaining three valences satisfied, independently of each other by any oligomeric, polymeric or copolymeric group, and wherein R^{I}O represents either a group that is a fragrance alkoxy group and/or biocide alkoxy group that is derived from the corresponding fragrance alcohol and/or biocide alcohol R^{I}OH, or wherein R^{I}O represents a group that is derived from an enolizable fragrance- and/or biocide ester, ketone or aldehyde, and wherein the agent additionally comprises at least one compound that is absorbed on hard and/or soft substrate surfaces, and that carries at least one cationic charge.

2. Agent according to Claim 1, **characterized in that** said agent is a fragrant, biocidal and/or fragrant biocidal agent.

3. Agent according to one of Claims 1-2, comprising at least one silicone oligomer, polymer or copolymer, which on hydrolysis releases a fragrant and/or biocidal alcohol, aldehyde, ketone or ester, which preferably has been incorporated by the reaction with an olefinic silane.

4. Agent according to Claim 3, **characterized in that** the olefinic silane is a reaction product of a fragrant and/or biocidal alcohol, aldehyde, ketone or ester with an olefinic halosilane or olefinic silicone alkoxide.

5. Agent according to one of Claims 3-4, **characterized in that** the olefinic silane is in accordance with Formula (2)
(R^{VIII}O)ₐ(R^{IX}O)_{b}(R^{X}O)_{c}(R^{XI})_{d} (R^{XII})ₑSiR^{XIII} (2)
wherein R^{VIII}O, R^{IX}O and R^{X}O, each independently of one another, represent fragrance alkoxy groups that derive from the corresponding fragrance alcohols R^{VIII}OH, R^{IX}OH and R^{X}OH,
wherein R^{XI}, R^{XII} are selected from the group of C₁₋₄₀ monovalent hydrocarbon groups and monovalent C₁₋₄₀ alkoxy groups, R^{XIII} is a C₂₋₄₀ monovalent unsaturated hydrocarbon group with an olefinic end group, and a has a value from 1-3, b, c, d, e have values from 0-2, with the proviso that a+b+c+d+e = 3 and a, b, c, d, e are integer numbers.

6. Agent according to one of Claims 3-4, **characterized in that** the olefinic silane is in accordance with Formula (3)
(R^{XIV})ₐ(R^{XV})_{b}(R^{XVI})_{c}(R^{XI})_{d} (R^{XII})ₑSiR^{XIII} (3)
wherein R^{XIV}, R^{XV} and R^{XVI} each independently of one another have the Formula (4) R^{XVII}(R^{XVIII})C=C(O-)R^{XIX} (4), R^{XVII}, R^{XVIII} and R^{XIX} independently of one another are chosen for each R^{XIV}, R^{XV} and R^{XVI}; R^{XI}, R^{XII} are selected from the group of C₁₋₄₀ monovalent hydrocarbon groups and monovalent C₁₋₄₀ alkoxy groups, R^{XIII} is a C₂₋₄₀ monovalent unsaturated hydrocarbon group with an olefinic end group, and a has a value from 1-3, b, c, d, e have values from 0-2, with the proviso that a+b+c+d+e = 3 and a, b, c, d, e are integer numbers, and R^{XVII}, R^{XVIII} und R^{XIX} are selected from the group consisting of hydrogen and monovalent C₁₋₁₀₀ hydrocarbon groups.

7. Agent according to Claim 6, **characterized in that** R^{XIV}, R^{XV} and R^{XVI} each independently of one another, possess the Formula (4) R^{XVII}(R^{XVIII})C=C(O-)-R^{XIX} (4) and derive from the group of the following aldehydes, ketones or esters, selected from 3-methyl-3(3-(1-methylethylphenyl))propanal), 2-methyl-3-(4-t-butylphenyl)propanal, 3-phenylpropional, 2-phenylpropional, propional, isobutyral, 2-methylbutyral, hexanal, octanal, nonanal, decanal, 3,7-dimethyl-1-al, p-tolylacetaldehyde, phenylacetaldehyde, 4-(3)(4-methyl-3-pentenyl)-3-cyclohexene-carbaldehyde, 2,6-dimethyl-5-heptenal, 3,7-dimethyl-2,6-octadienal, trans-4-decenal, cyclamen aldehyde, 4-(p-methoxyphenyl)-2-butanone, acetophenone, 2-pentanone, 2-butanone, 2-heptanone, 3-heptanone, 2-decanone, 3-penten-2-one, 6-methyl-5-hepten-2-one, geranyl acetone, 5-methyl-alpha-ionone, 2-acetonaphtone, 2-methyl-3-phenylpropan-2-yl acetate, linalyl acetate, menthanyl acetate, 2-phenylethyl acetate, tetrahydrolinalyl acetate, phenethyl propionate, phenethyl hexanoate, butyl acetate, phenoxyethyl isobutyrate, p-tert.-butylcyclohexyl acetate, linalyl acetate, dimethylbenzyl carbinyl acetate (DMBCA), phenylethyl acetate, benzyl acetate, ethylmethylphenyl glycinate, allylcyclohexyl propionate, styrallyl propionate, benzyl salicylate, cyclohexyl salicylate, floramate, melusate and jasmacyclate, linear alkanals having 8-18 carbon atoms, citral, citronellal, citronellyloxy-acetaldehyd, cyclamen aldehyd, lilial and bourgeonal, the ionones, α-isomethylionone and methyl cedryl ketone.

8. Agent according to Claim 7, **characterized in that** the fragrance alkoxy groups and/or biocide alkoxy groups R^{VIII}O, R^{IX}O and R^{X}O are each derived from fragrance alcohols and/or biocide alcohols, selected from the group 2-methylbutanol, 3-pentanol, n-pentanol, 2-pentanol, n-hexanol, 2-methylpentanol, 1-decanol, sandela, nonadol, dimetol, thymol, 1-heptanol, menthol, eugenol, vanillin, o-vanillin, 4-(p-hydroxyphenyl)-2-butanone, syringe aldehyde, prenol, *cis*-3-hexanol, *trans*-3-hexanol, *cis*-4-heptenol, *trans*-2-octenol, *trans*-2-*cis*-6-nonadienol, nerol, ebanol, crotyl alcohol, oleyl alcohol, linalool, α-terpineol, β-phenethyl alcohol, cinnamyl alcohol, benzyl alcohol, α-methylbenzyl alcohol, nonyl alcohol, 1-octanol, 3-octanol, phenethyl salicylate, hydrocinnamyl alcohol, *cis*-6-nonen-1-ol, *trans*-2-nonen-1-ol, methyl salicylate, *cis*-3-octenol, anisyl alcohol, carvacrol, dihydrocarveol, benzyl salicylate, tetrahydrogeraniol, ethyl salicylate, ethyl vanillin, isoeugenol, isopulegol, lauryl alcohol, tetrahydrolinalool, 2-phenoxyethanol, citronellol, farnesol, and geraniol.

9. Agent according to one of Claims 1-8 **characterized in that** the molecular weight of the oligomer, polymer or copolymer is up to about 300 000, preferably up to 100 000, particularly preferably in the range of about 150 to about 30 000.

10. Agent, according to one of Claims 1-9, **characterized in that** the content of the fragrance group or biocide group in the total weight of the oligomer, polymer or copolymer is up to 80 wt.%, preferably up to 70 wt.%, advantageously up to 70 wt.%, particularly between 0.001 and 60 wt.%, each based on the total agent.

11. Agent, according to one of Claims 1-10, **characterized in that** the oligomer, polymer or copolymer is essentially unbranched, preferably to at least 50 %, advantageously to at least 60 %, in particular to at least 70 % linear.

12. Agent according to one of Claims 1-11, **characterized in that** the oligomer, polymer copolymer conforms to Formula (5).
M_{f}M^{F}gDₕO^{F}iTⱼT^{F}ₖQₗ (5)
with M: R^{XX}R^{XXI}R^{XXII}SiO_{1/2}; M^{F}: R^{XX}RX^{XXI}R^{F}SiO_{1/2}; D: R^{XXIII}R^{XXIV}SiO_{2/2}; D^{F}: R^{XXIII}R^{F}SiO_{2/2}; T: R^{XXV}SiO_{3/2}; T^{F}: R^{F}SiO_{3/2} Q: SiO_{4/2}, wherein
R^{XX}, R^{XXI}, R^{XXII}, R^{XXIII}, R^{XXIV}, R^{XXV} each independently of one another, are selected for each M, M^{F}, D, D^{F}, T and T^{F}, from the group of C₁₋₄₀ monovalent, straight chain or branched, saturated or unsaturated alkyl or alkoxy groups or from the group of C₁₋₄₀ monovalent aryl or aryloxy groups, wherein the above-mentioned alkyl, alkoxy, aryl, aryloxy groups can be substituted, and can comprise heteroatoms such as oxygen, nitrogen, sulfur or halogens or others, wherein f, g are positive numbers, h, i, j, k, l are positive numbers or equal to zero, wherein at least one of h, i, j, k, l is not equal to zero and at least one of g, i, or k equals 1 or is greater than 1, and
wherein R^{F} is derived from one of the abovementioned groups (R^{VIII}O)ₐ(R^{IX}O)_{b}(R^{X}O)_{c}(R^{XI})_{d}(R^{XI})_{d}SiR^{XIII} (according to Formula (2)) and/or (R^{XIV})ₐ(R^{XV})_{b}(R^{XVI})_{c}(R^{XI})_{d}(R^{XII})ₑSiR^{XIII} (according to Formula (3)), wherein this group R^{F} is bonded through a bivalent C₂₋₄₀ hydrocarbon bridging member, derived from R^{XIII} (a C₂₋₄₀ monovalent unsaturated hydrocarbon group with an olefinic end group) with a Si-atom of the oligomer, polymer or copolymer.

13. Agent according to one of Claims 1-12, **characterized in that** the oligomer, polymer copolymer is selected from the following Formulae: 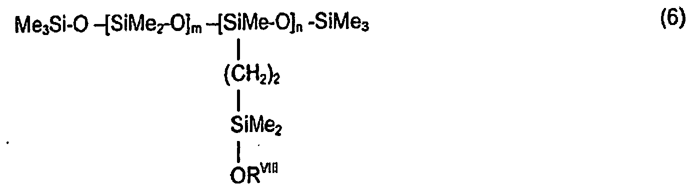 wherein OR^{VIII} stands for a fragrance alkoxy group or biocide alkoxy group, particularly for a phenylethyl alcohol group, and wherein m and n each have a positive value, with the limitation that the resulting silicone reaches a molecular weight of at least 150
and/or
R^{VIII}O-SiMe₂-(CH₂)₂-[SiMe₂-O]ₚ-SiMe₂-(CH₂)₂-SiMe₂-OR^{VIII} (7)
wherein OR^{VIII} stands for a fragrance alkoxy group or biocide alkoxy group, particularly for a phenylethyl alcohol group, with p greater than 0, with the limitation that the resulting silicone reaches a molecular weight of at least 150.

14. Agent according to one of Claims 1-13, **characterized in that** the oligomer, polymer or copolymer is comprised in the agent in amounts of greater than 0.001 wt.%, preferably from 0.002 to 10 wt.%, particularly from 0.01 to 5 wt.%, particularly preferably from 0.02 to 3 wt.% and quite particularly preferably in quantities of 0.05 to 2 wt.%, in each case based on the total agent.

15. Agent according to one of Claims 1-14, **characterized in that** the compound that carries at least one cationic charge and is absorbed on hard and/or soft surfaces of substrates is preferably comprised in amounts of greater than 0.01 wt.%, particularly from 0.02 to 45 wt.%, advantageously in quantities of 5 to 40 wt.%, particularly advantageously from 10-35 wt.%, in each case based on the total agent.

16. Agent according to one of Claims 1-15, **characterized in that** the compound that carries at least one cationic charge and is absorbed on hard and/or soft surfaces of substrates is a compound that is selected from the group of cationic or amphoteric emulsifiers, cationic surfactants, zwitterionic compounds, ampholytes, amphosurfactants, betaines and/or cationic or amphoteric polymers.

17. Agent according to one of Claims 1-16, **characterized in that** the compound that is absorbed on hard and/or soft surfaces of substrates, is a compound that in aqueous media at pH-values below 4, preferably below 5, advantageously below 6, particularly advantageously below 7, quite particularly advantageously below 8, most advantageously below 9, especially below 10, possesses at least one cationic charge, the pH being measured at 20 °C.

18. Agent according to one of Claims 1-17, **characterized in that** the compound that is absorbed on hard and/or soft surfaces of substrates is quaternary ammonium compounds, advantageously alkylated quaternary ammonium compounds, in which at least one alkyl chain is interrupted by an ester group and/or amido group.

19. Agent according to one of Claims 1-18, **characterized in that** the compound that is absorbed on hard and/or soft surfaces of substrates is a quaternary ammonium compound selected from the following Formulae (9): 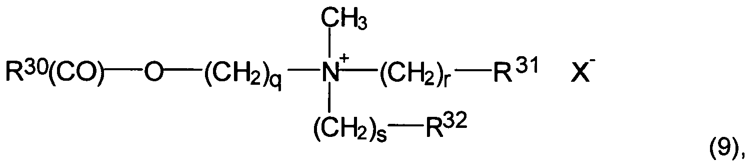 wherein, R³⁰ stands for an aliphatic alkyl group with 12 to 22 carbon atoms and 0, 1, 2 or 3 double bonds, R³¹ stands for H, OH or in particular O(CO)R^{a}, R³² independently of R³¹ stands for H, OH or O(CO)R^{b}, wherein R^{a} and R^{b}, independently of each other, each stand for an aliphatic alkyl group having 12 to 22 carbon atoms with 0, 1, 2 or 3 double bonds, q, r and s independently of each other can each have the value 1, 2 or 3, X- is an adequate anion, preferably a halide, methosulfate, methophosphate or phosphate ion as well as mixtures thereof,
and/or from the Formula (12): 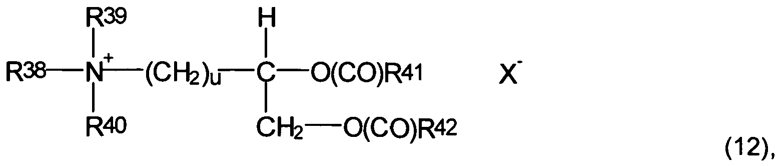 wherein R³⁸, R³⁹ and R⁴⁰ independently of one another stand for a 6₁₋₄ alkyl, alkenyl or hydroxyalkyl group, R⁴¹ and R⁴², each independently selected, represents a C₈₋₂₈ alkyl group with 0,1, 2 or 3 double bonds and u is a number between 0 and 5, X- is an adequate anion, preferably a halide, methosulfate, methophosphate or phosphate ion as well as mixtures thereof.

20. Agent according to one of Claims 1-19, **characterized in that** the compound that is absorbed on hard and/or soft surfaces of substrates is N-methyl-N(2-hydroxyethyl)-N,N-(ditallow acyloxyethyl) ammonium methosulfate or N-methyl-N(2-hydroxyethyl)-N,N-(dipalmitoylethyl) ammonium methosulfate.

21. Agent according to one of Claims 1-20, **characterized in that** the compound that is absorbed on hard and/or soft surfaces of substrates and which preferably carries at least one cationic charge is a zwitterionic compound according to Formula (18) 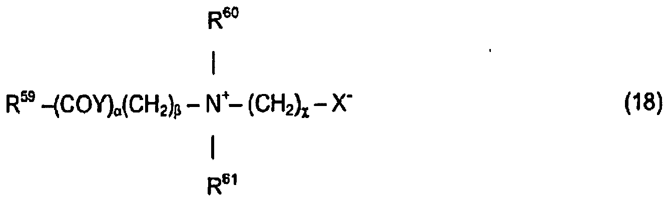 in which R⁵⁹ stands for a C₆₋₂₈ alkyl or alkenyl group, and in which R⁶⁰ and R⁶¹ are each, independently of one another, C₁₋₄ alkyl groups; and in which α stands for the number 0 or 1, β and X are each selected independently of one another from whole numbers from 1 to 4 and in which Y is oxygen or nitrogen and in which X is a compatible anion.

22. Agent according to one of Claims 1-21, **characterized in that** the compound that is absorbed on hard and/or soft surfaces of substrates and which preferably carries at least one cationic charge is an alkylamido alkylene dimethylcarboxylic acid-betaine according to Formula (19): 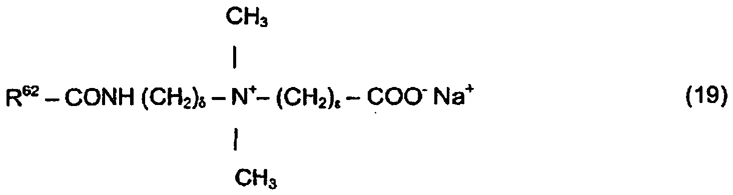 wherein δ und ε, independently of one another, are integer numbers from 1-4, advantageously δ is equal to 2 or 3 and ε equals 2 or 3 and R⁶² stands for a C₁₀₋₁₈ alkyl chain or mixtures thereof.

23. Agent according to one of Claims 1-22, **characterized in that** the compound that is absorbed on hard and/or soft surfaces of substrates and which carries at least one cationic charge is a cationic nitrile according to Formula (14): 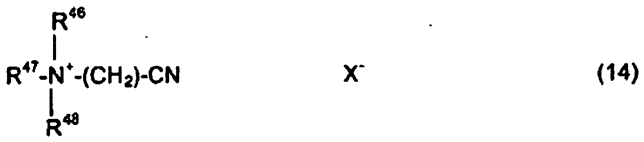 in which R⁴⁶ stands for -H, -CH₃, a C₂₋₂₄ alkyl or alkenyl group, a substituted C₂₋₂₄ alkyl or alkenyl group having at least one substituent from the group of -Cl, -Br, -OH, -NH₂, -CN, an alkylaryl or alkenylaryl group having a C₁₋₂₄ alkyl group or for a substituted alkylaryl or alkenylaryl group having a C₁₋₂₄ alkyl group and at least a further substituent on the aromatic ring, R⁴⁷ and R⁴⁸, independently of one another are selected from -CH₂-CN, -CH₃, -CH₂-CH₃, -CH₂-CH₂-CH₃, -CH(CH₃)-CH₃, -CH₂-OH, -CH₂-CH₂-OH, -CH(OH)-CH₃, -CH₂-CH₂-CH₂-OH, -CH₂-CH(OH)-CH₃, - CH(OH)-CH₂-CH₃, -(CH₂CH₂-O)ₙH with n equal to 1, 2, 3, 4, 5 or 6 and X is an anion.

24. Agent according to one of Claims 1-23, **characterized in that** it comprises at least one cationic nitrile according to Formula (15). 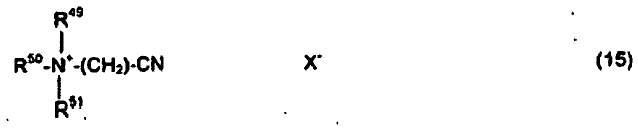 in which R⁴⁹, R⁵⁰ und R⁵¹ independently of one another are selected from -CH₃, -CH₂-CH₃, -CH₂-CH₂-CH₃, -CH(CH₃)-CH₃, wherein R⁴⁹ can also be -H and X is an anion, wherein preferably R⁵⁰ = R⁵¹ = -CH₃ and in particular R⁴⁹ = R⁵⁰ = R⁵¹ = -CH₃ and compounds of the formulae (CH₃)₃N⁽⁺⁾CH₂-CN X⁻, (CH₃CH₂)₃N(⁺)CH₂-CN X⁻ , (CH₃CH₂CH₂)₃N⁽⁺⁾CH₂-CN X⁻, (CH₃CH(CH₃))₃N⁽⁺⁾CH₂-CN X⁻, or (HO-CH₂-CH₂)₃N⁽⁺⁾CH₂-CN X⁻ are particularly preferred.

25. Agent according to one of Claims 23-24, **characterized in that** X- stands for an anion that is selected from the group chloride, bromide, iodide, hydrogen sulfate, methosulfate, lauryl sulfate, dodecylbenzene sulfonate, p-toluene sulfonate (tosylate), cumene sulfonate or xylene sulfonate or mixtures thereof.

26. Agent according to one of Claims 1-25, **characterized in that** the agent is present in solid, dispersed, powder, granular or compressed form, with the proviso that in the compressed form, it is particularly in the form of a tablet that consists of one or a plurality of phases.

27. Agent according to one of Claims 1-25, **characterized in that** the agent is present in a gel or in liquid form, particularly emulsified, wherein advantageously it comprises up to 95 wt.%, preferably 20 to 90 wt.% and particularly preferably 50 to 80 wt.% of one or a plurality of solvents.

28. Agent according to one of Claims 1-27, **characterized in that** the agent comprises at least one further substance typically comprised in detergents or cleaning agents, preferably a substance from the group of surfactants, builders (inorganic and organic builders), bleaching-agents, bleach activators, bleach stabilizers, bleach catalysts, enzymes, special polymers (for example those with cobuilder properties), graying inhibitors, optical brighteners, UV-protection agents, soil repellents, electrolytes, colorants, perfumes, fragrances, perfume carriers, pH adjustors, chelating agents, fluorescence agents, foam inhibitors, anti-creasing agents, antioxidants, quaternary ammonium compounds, antistatics, ironing auxiliaries, UV-absorbers, antiredeposition agents, germicides, antimicrobials, fungicides, viscosity regulators, pearlizers, color-transfer inhibitors, anti-shrinkage agents, corrosion inhibitors, conservation agents, softeners, softening rinse agents, protein hydrolyzates, water-proofing and impregnation agents, hydrotropes, silicone oils as well as swelling agents and non-slip agents.

29. Use of an agent according to one of the previous Claims 1 to 28 as a cosmetic formulation.

30. Use of an agent according to one of the previous Claims 1 to 28 as a textile treatment agent, particularly as a detergent or post-treatment agent or rinsing agent or as a softening rinse agent.

31. Use of an agent according to one of the previous Claims 1 to 28 for conditioning keratinous fibers.

32. Process for treating hard and/or soft substrate surfaces **characterized in that** an effective quantity of an agent according to one of the Claims 1-28 is applied to the substrate to be treated by means of a spray dispenser, with the proviso that the agent is present in liquid form, particularly emulsified.

33. Use of an agent according to one of the previous Claims 1 to 28 for cleaning and/or caring for hard surfaces.

34. Product, comprising an agent according to one of the Claims 1-28 and a spray dispenser.

35. Conditioning substrate, **characterized in that** the substrate is impregnated and/or coated with an agent according to one of the Claims 1-28.

36. Conditioning substrate according to Claim 35, **characterized in that** the substrate consists of a fleece material, particularly a viscose fleece.

37. Conditioning substrate according to one of Claims 35-36, **characterized in that** the substrate has a grammage between 20 to 1000 g/m², particularly from 30 to 500 g/m².

38. Conditioning substrate according to one of Claims 35-37, **characterized in that** the substrate has a size of 0.2 to 0.005 m².

39. Process for conditioning textiles, **characterized in that** one or a plurality of conditioning substrates according to Claims 35-38 are used in a textile-drying process.

40. Use of a compound that carries at least one cationic charge and which is absorbed on hard and/or soft surfaces of substrates, to fix an oligomer, polymer or copolymer that comprises the following structural element shown in Formula (1) at least once, on hard and/or soft substrate surfaces: 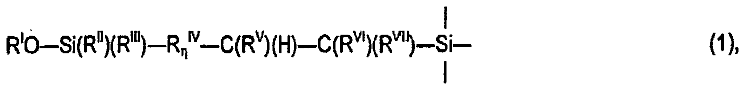 wherein R^{II}, R^{III}, independently of one another each stand for an aliphatic or aromatic, linear or branched, saturated or unsaturated, substituted or unsubstituted hydrocarbon group that respectively can comprise heteroatoms such as oxygen, nitrogen, sulfur or halogens or others and wherein R_{η}^{IV} stands for a carbon bridging member that is an aliphatic or aromatic, linear or branched, saturated or unsaturated, substituted or unsubstituted hydrocarbon group that respectively can comprise heteroatoms such as oxygen, nitrogen, sulfur or halogens or others, wherein the number η is 0 to 10, and wherein R^{V}, R^{VI}, R^{VII}, independently of each other each stand for hydrogen or an aliphatic or aromatic, linear or branched, saturated or unsaturated, substituted or unsubstituted hydrocarbon group that respectively can comprise heteroatoms such as oxygen, nitrogen, sulfur or halogens or others, and wherein the terminal silicon in Formula 1 has its remaining three valences satisfied, independently of each other by any oligomeric, polymeric or copolymeric group, and wherein R^{I}O represents either a group that is a fragrance alkoxy group and/or biocide alkoxy group that is derived from the corresponding fragrance alcohol and/or biocide alcohol R^{I}OH, or wherein R^{I}O represents a group that is derived from an enolizable fragrance- and/or biocide ester, ketone or aldehyde.

## Revendications

1. Agent, en particulier agent de lavage ou de nettoyage ou agent de conditionnement ou agent cosmétique, comprenant au moins un oligomère, un polymère ou un copolymère, qui contient à raison d'au moins une fois un élément de structure répondant à la formule (1) 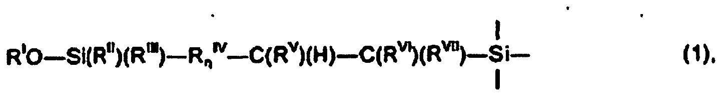 dans laquelle R^{II}, R^{III} représentent respectivement, indépendamment l'un de l'autre, un résidu d'hydrocarbure aliphatique ou aromatique, à chaîne droite ou ramifiée, saturé ou insaturé, substitué ou non substitué, qui peut contenir respectivement des hétéroatomes tels qu'un atome d'oxygène, un atome d'azote, un atome de soufre ou un atome d'halogène ou analogue, et dans laquelle R_{η}^{IV} représente un membre de pont carbone qui représente un résidu d'hydrocarbure aliphatique ou aromatique, à chaîne droite ou ramifiée, saturé ou insaturé, substitué ou non substitué, qui peut contenir respectivement des hétéroatomes tels qu'un atome d'oxygène, un atome d'azote, un atome de soufre ou un atome d'halogène ou analogue, le numéro d'ordre η s'élevant de 0 à 10, et dans laquelle R^{V}, R^{VI}, R^{VII} représentent respectivement, indépendamment l'un de l'autre, un atome d'hydrogène ou un résidu d'hydrocarbure aliphatique ou aromatique, à chaîne droite ou ramifiée, saturé ou insaturé, substitué ou non substitué, qui peut contenir respectivement des hétéroatomes tels qu'un atome d'oxygène, un atome d'azote, un atome de soufre ou un atome d'halogène ou analogue, et dans laquelle le silicium terminal dans la formule (1) présente, à ses trois valences restantes, indépendamment l'une de l'autre, n'importe quel résidu de l'oligomère, du polymère ou du copolymère, et dans laquelle R^{I}O représente un résidu à savoir un groupe alcoxy de substance aromatisante et/ou un groupe alcoxy de biocide qui dérive de l'alcool de substance aromatisante et/ou de biocide R^{I}OH correspondant, ou bien R^{I}O représente un résidu qui dérive d'un ester, d'une cétone ou d'un aldéhyde énolisable de substance aromatisante et/ou de biocide, et l'agent comprenant en outre au moins un composé se fixant sur des surfaces de substrats dures et/ou souples, qui porte au moins une charge cationique.

2. Agent selon la revendication 1, **caractérisé en ce qu'**il s'agit d'un agent aromatisant, d'un agent biocide et/ou d'un agent biocide aromatisant.

3. Agent selon l'une quelconque des revendications 1 à 2, comprenant au moins un oligomère, un polymère ou un copolymère de silicone, qui libère, lors de l'hydrolyse un alcool, un aldéhyde, une cétone ou un ester aromatisant et/ou biocide, qui a été introduit de préférence par mise en réaction avec un silane oléfinique.

4. Agent selon la revendication 3, **caractérisé en ce que** le silane oléfinique est un produit réactionnel d'un alcool, d'un aldéhyde, d'une cétone ou d'un ester aromatisant et/ou biocide et d'un halogénosilane oléfinique ou d'un alcoxyde de silicium oléfinique.

5. Agent selon l'une quelconque des revendications 3 à 4, **caractérisé en ce qu'**il s'agit d'un silane oléfinique répondant à la formule (2)
**(R^{VIII}O)ₐ(R^{IX}O)_{b}(R^{X}O)_{c}(R^{XI})_{d} (RXII)ₐSiR^{XIII}** (2)
dans laquelle R^{VIII}O, R^{IX}O et R^{X}O représentent respectivement, indépendamment l'un de l'autre, des groupes alcoxy de substance aromatisante qui dérivent des alcools correspondants de substances aromatisantes R^{VIII}OH, R^{IX}OH et R^{X}OH, R^{XI}, R^{XII} étant choisis parmi le groupe comprenant des résidus d'hydrocarbures monovalents en C₁-C₄₀, et de résidus alcoxy monovalents en C₁-C₄₀, R^{XIII} représentant un résidu d'hydrocarbure insaturé monovalent en C₂-C₄₀ comprenant un groupe terminal oléfinique, et a possède une valeur de 1 à 3, b, c, d, e possédant des valeurs de 0 à 2, avec cette mesure que a+b+c+d+e = 3 et que a, b, c, d, e représentent des nombres entiers.

6. Agent selon l'une quelconque des revendications 3 à 4, **caractérisé en ce qu'**il s'agit d'un silane oléfinique répondant à la formule (3)
**(R^{XIV})ₐ(R^{XV})_{b}(R^{XVI})_{c}(R^{XI})_{d}(R^{XII})ₑSiR^{XIII}** (3)
dans laquelle R^{XIV}, R^{XV} et R^{XVI} présentent respectivement, indépendamment l'un de l'autre, la formule (4) R^{XVII}(R^{XVIII})C=C(O-)R^{XIX} (4), R^{XVII}, R^{XVIII} et R^{XIX} étant choisis, indépendamment l'un de l'autre, pour chaque résidu R^{XIV}, R^{XV} et R^{XVI}, R^{XI}, R^{XII} étant choisis parmi le groupe comprenant des résidus d'hydrocarbures monovalents en C₁-C₄₀, et de résidus alcoxy monovalents en C₁-C₄₀, R^{XIII} représentant un résidu d'hydrocarbure insaturé monovalent en C₂-C₄₀ comprenant un groupe terminal oléfinique, et a possède une valeur de 1 à 3, b, c, d, e possédant des valeurs de 0 à 2, avec cette mesure que a+b+c+d+e = 3 et que a, b, c, d, e représentent des nombres entiers, et R^{XVII}, R^{XVIII} et R^{XIX} sont choisis parmi le groupe constitué par un atome d'hydrogène et des résidus d'hydrocarbures monovalents en C₁-C₁₀₀.

7. Agent selon la revendication 6, dans lequel R^{XIV}, R^{XV} et R^{XVI} présentent respectivement, indépendamment l'un de l'autre, la formule (4) R^{XVII}(R^{XVIII})C=C(O-)R^{XIX} (4) et dérive du groupe des aldéhydes, des cétones ou des esters ci-après choisis parmi le 3-méthyl-3-(3-(1-méthyléthylphényl))propanal, le 2-méthyl-3-(4-tert-butylphényl)propanal, le 3-phénylpropional, le 2-phénylpropional, le propional, l'isobutyral, le 2-méthylbutyral, l'hexanal, l'octanal, le nonanal, le décanal, le 3,7-diméthyl-1-al, le p-tolylacétaldéhyde, le phénylacétaldéhyde, le 4-(3)-(4-méthyl-3-pentényl)-3-cyclohexène-carbaldéhyde, le 2,6-diméthyl-5-hepténal, le 3,7-diméthyl-2,6-octadiénal, le trans-4-décénal, le cyclamenaldéhyde, la 4-(p-méthoxyphényl)-2-butanone, l'acétophénone, la 2-pentanone, la 2-butanone, la 2-heptanone, la 3-heptanone, la 2-décanone, la 3-pentén-2-one, la 6-méthyl-5-heptén-2-one, la géranylacétone, la 5-méthyl-alpha-ionone, la 2-acétonaphtone, l'acétate de 2-méthyl-3-phénylpropan-2-yle, l'acétate de linalyle, l'acétate de menthanyle, l'acétate de 2-phényléthyle, l'acétate de tétrahydrolinalyle, le propionate de phénétyle, l'hexanoate de phénétyle, l'acétate de butyle, l'isobutyrate de phénoxyéthyle, l'acétate de p-tert.-butylcyclohexyle, l'acétate de linalyle, l'acétate de diméthylbenzylcarbinyle (DMBCA), l'acétate de phényléthyle, l'acétate de benzyle, le glycinate d'éthylméthylphényle, le propionate d'allylcyclohexyle, le propionate de styrylallyle, le salicylate de benzyle, le salicylate de cyclohexyle, le floramate, le mélusate et le jasmacyclate, des alcanals linéaires contenant de 8 à 18 atomes de carbone, le citral, le citronnellal, l'oxyacétaldéhyde de citronellyle, le cyclamenaldéhyde, le lilial, le bourgeonal, les ionones, l'α-isométhylionone et la méthylcédrylcétone.

8. Agent selon la revendication 7, **caractérisé en ce que** les groupes alcoxy R^{VIII}O, R^{IX}O et R^{X}O de substances aromatisantes et/ou de biocides dérivent respectivement d'alcools de substances aromatisantes et/ou de biocides choisis parmi le 2-méthylbutanol, le 3-pentanol, le n-pentanol, le 2-pentanol, le n-hexanol, le 2-méthylpentanol, le 1-décanol, le Sandela, le nonadol, le dimétol, le thymol, le 1-heptanol, le menthol, l'eugénol, la vanilline, la o-vanilline, la 4-(p-hydroxyphényl)-2-butanone, l'aldéhyde de syringa, le prenol, le cis-3-hexanol, le trans-3-hexanol, le cis-4-hepténol, le trans-2-octénol, le trans-2-cis-6-nonadiénol, le nérol, l'ébanol, l'alcool crotylique, l'alcool oléylique, le linalol, l'α-terpinéol, l'alcool β-phénéthylique, l'alcool cinnamique, l'alcool benzylique, l'alcool α-méthylbenzylique, l'alcool nonylique, le 1-octanol, le 3-octanol, le salicylate de phénétyle, l'alcool hydrocinnamique, le cis-6-nonén-1-ol, le trans-2-nonén-1-ol, le salicylate de méthyle, le cis-3-octénol, l'alcool anisylique, le carvacrol, le dihydrocarvéol, le salicylate de benzyle, le tétrahydrogéraniol, le salicylate d'éthyle, l'éthylvanilline, l'isoeugénol, l'isopulégol, l'alcool laurylique, l'alcool tétrahydrolinalylique, le 2-phénoxyéthanol, le citronnellol, le farnésol et le géraniol.

9. Agent selon l'une quelconque des revendications 1 à 8, **caractérisé en ce que** la masse molaire de l'oligomère, du polymère ou du copolymère s'élève jusqu'à environ 300 000, de préférence jusqu'à 100 000, de manière particulièrement préférée se situe dans la plage d'environ 150 à environ 30 000.

10. Agent selon l'une quelconque des revendications 1 à 9, **caractérisé en ce que** la fraction du résidu de substance aromatisante respectivement du résidu biocide, par rapport à la masse totale de l'oligomère, du polymère ou du copolymère représente jusqu'à 80 % en poids, de préférence jusqu'à 70 % en poids, et se situe en particulier entre 0,001 et 60 % en poids, chaque fois rapportés à l'agent dans son ensemble.

11. Agent selon l'une quelconque des revendications 1 à 10, **caractérisé en ce que** l'oligomère, le polymère ou le copolymère est essentiellement non ramifié, de préférence est linéaire à concurrence d'au moins 50 %, de manière avantageuse à concurrence d'au moins 60 %, en particulier à concurrence d'au moins 70 %.

12. Agent selon l'une quelconque des revendications 1 à 11, **caractérisé en ce que** l'oligomère, le polymère ou le copolymère répond à la formule (5) :
**M_{f}M^{F}_{g}DₕD^{F}ₗTⱼT^{F}ₖOₗ** (5)
dans laquelle M représente R^{XX}R^{XXI}R^{XXII}SiO_{1/2}; M^{F} représente R^{XX}R^{XXI}R^{F}SiO_{1/2} ; D représente R^{XXIII}R^{XXIV}SiO_{2/2}; D^{F} représente R^{XXIII}R^{F}SiO_{2/2}; T représente R^{XXV}SiO_{3/2}; T^{F} représente R^{F}SiO_{3/2} ; Q représente SiO_{4/2}; où R^{XX}, R^{XXI}, R^{XXII}, R^{XXIII}, R^{XXIV}, R^{XXV} sont choisis respectivement, indépendamment l'un de l'autre pour chaque M, M^{F}, D, D^{F}, T et T^{F} parmi le groupe comprenant des résidus alkyle ou alcoxy monovalents en C₁-C₄₀, à chaîne droite ou ramifiée, saturés ou insaturés, ou des résidus aryle ou aryloxy monovalents en C₁-C₄₀, les résidus alkyle, alcoxy, aryle, aryloxy susmentionnés pouvant être substitués, et pouvant également contenir des hétéroatomes tels qu'un atome d'oxygène, un atome d'azote, un atome de soufre ou un atome d'halogène ou analogue, f, g représentant des nombres positifs, h, i, j, k, l représentant des nombres positifs ou étant égaux à zéro, au moins un des indices h, i, j, k, l n'étant pas égal à zéro et au moins un des indices g, i ou k étant égal à 1 ou supérieur à 1, et dans laquelle R^{F} dérive d'un des résidus susmentionnés (R^{VIII}O)ₐ(R^{IX}O)_{b}(R^{X}O)_{c}(R^{XI})_{d}(R^{XII})ₑSiR^{XIII} (conformément à la formule (2)) et/ou (R^{XIV})ₐ(R^{XV})_{b}(R^{XVI})_{c}(R^{XI})_{d}(R^{XII})ₑSiR^{XIII} (conformément à la formule (3)), ce résidu R^{F} étant lié, via un membre faisant pont à base d'un hydrocarbure bivalent en C₂-C₄₀, qui dérive de R^{XIII} (un résidu d'hydrocarbure monovalent insaturé en C₂-C₄₀ comprenant un groupe terminal oléfinique), à un atome de silicium de l'oligomère, du polymère ou du copolymère.

13. Agent selon l'une quelconque des revendications 1 à 12, **caractérisé en ce que** l'oligomère, le polymère ou le copolymère est choisi parmi les formules suivantes : 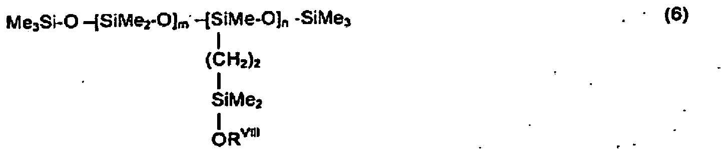 dans laquelle OR^{VIII} représente un résidu alcoxy d'une substance aromatisante ou d'un biocide, en particulier un résidu d'alcool phényléthylique, m et n possédant respectivement une valeur positive, avec cette mesure que la silicone résultant atteigne une masse molaire d'au moins 150 ;
et/ou
**R^{VIII}O-SiMe₂-(CH₂)₂-[SiMe₂-O]ₚ-SiMe₂-(CH₂)₂-SiMe₂-OR^{VIII}** (7)
dans laquelle OR^{VIII} représente un résidu alcoxy de substance aromatisante ou de biocide, en particulier un résidu d'alcool phényléthylique, p étant supérieur à 0, avec cette mesure que la silicone résultant atteigne une masse molaire d'au moins 150.

14. Agent selon l'une quelconque des revendications 1 à 13, **caractérisé en ce que** l'oligomère, le polymère ou le copolymère est contenu dans des quantités supérieures à 0,001 % en poids, de manière avantageuse dans des quantités de 0,002 à 10 % en poids, en particulier de 0,01 à 5 % en poids, de manière particulièrement préférée de 0,02 à 3 % en poids, et de manière tout particulièrement préférée dans des quantités de 0,05 à 2 % en poids, chaque fois rapportés à l'agent dans sa totalité.

15. Agent selon l'une quelconque des revendications 1 à 14, **caractérisé en ce qu'**il contient le composé se fixant sur des surfaces de substrats dures et/ou souples, qui porte au moins une charge cationique, dans des quantités supérieures à 0,01 % en poids, en particulier dans des quantités de 0,02 à 45 % en poids, de manière avantageuse de 5 à 40 % en poids, de manière particulièrement avantageuse de 10 à 35 % en poids, chaque fois rapportés à l'agent dans sa totalité.

16. Agent selon l'une quelconque des revendications 1 à 15, **caractérisé en ce que**, en ce qui concerne le composé se fixant sur des surfaces de substrats dures et/ou souples, qui porte au moins une charge cationique, il s'agit d'un composé qui est choisi parmi le groupe comprenant des émulsifiants cationiques ou amphotères, des agents tensioactifs cationiques, des composés zwitterioniques, des ampholytes, des amphotensioactifs, de bétaïnes et/ou d'autres polymères cationiques ou amphotères.

17. Agent selon l'une quelconque des revendications 1 à 16, **caractérisé en ce que**, en ce qui concerne le composé se fixant sur des surfaces de substrats dures et/ou souples, il s'agit d'un composé qui, dans un milieu aqueux, à des valeurs de pH inférieures à 4, de préférence inférieures à 5, de manière avantageuse inférieures à 6, de manière particulièrement avantageuse inférieures à 7, de manière tout particulièrement avantageuse inférieures à 8, de manière encore plus particulièrement avantageuse inférieures à 9, en particulier inférieures à 10, présente au moins une charge cationique, la valeur de pH étant mesurée à 20 °C.

18. Agent selon l'une quelconque des revendications 1 à 17, **caractérisé en ce que**, en ce qui concerne le composé se fixant sur des surfaces de substrats dures et/ou souples, il s'agit de composés d'ammonium quaternaires, de préférence de composés d'ammonium quaternaires alkylés, dont au moins une chaîne alkyle est interrompue par un groupe ester et/ou par un groupe amido.

19. Agent selon l'une quelconque des revendications 1 à 18, **caractérisé en ce que**, en ce qui concerne le composé se fixant sur des surfaces de substrats dures et/ou souples, il s'agit d'un composé d'ammonium quaternaire choisi parmi les formules suivantes (9) : 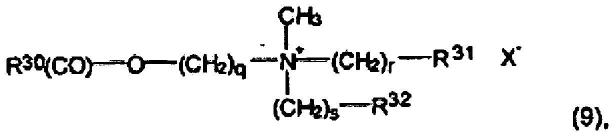 dans laquelle R³⁰ représente un résidu alkyle aliphatique contenant de 12 à 22 atomes de carbone avec 0, 1, 2 ou 3 liaisons doubles ; R³¹ représente un atome d'hydrogène, un groupe OH ou en particulier un groupe O(CO)R^{a} ; R³² représente, indépendamment de R³¹, un atome d'hydrogène, un groupe OH ou un groupe O(CO)R^{b} ; R^{a} et R^{b} représentent respectivement, indépendamment l'un de l'autre, un résidu alkyle aliphatique contenant de 12 à 22 atomes de carbone avec 0, 1, 2 ou 3 liaisons doubles ; q, r et s peuvent prendre respectivement, indépendamment l'un de l'autre, la valeur 1, 2 ou 3 ; X- représente un anion approprié, de préférence un ion d'halogénure, un ion de méthosulfate, un ion de méthophosphate ou un ion de phosphate, ainsi que des mélanges desdits ions ;
et/ou (12) 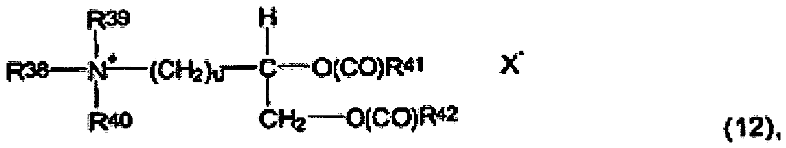 dans laquelle R³⁸, R³⁹ et R⁴⁰ représentent, indépendamment l'un de l'autre, un groupe alkyle en C₁-C₄, un groupe alcényle ou un groupe hydroxyalkyle ; R⁴¹ et R⁴² représentent respectivement, indépendamment l'un de l'autre, un groupe alkyle en C₈-C₂₈ comprenant 0, 1, 2 ou 3 liaisons doubles et u représente un nombre entre 0 et 5 ; X- représente un anion approprié, de préférence un ion d'halogénure, un ion de méthosulfate, un ion de méthophosphate ou un ion de phosphate, ainsi que des mélanges desdits ions.

20. Agent selon l'une quelconque des revendications 1 à 19, **caractérisé en ce que**, en ce qui concerne le composé se fixant sur des surfaces de substrats dures et/ou souples, il s'agit du méthosulfate de N-méthyl-N(2-hydroxyéthyl)-N,N-(disuifacyloxyéthyl)ammonium ou du méthosulfate de N-méthyl-N(2-hydroxyéthyl)-N,N-(dipalmitoyléthyl)ammonium.

21. Agent selon l'une quelconque des revendications 1 à 20, **caractérisé en ce que**, en ce qui concerne le composé se fixant sur des surfaces de substrats dures et/ou souples, qui porte au moins une charge cationique, il s'agit d'un composé zwitterionique répondant à la formule (18) : 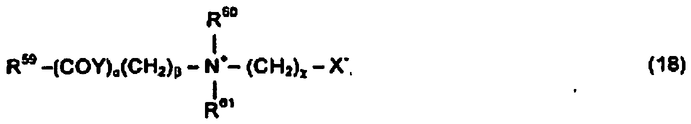 dans laquelle R⁵⁹ représente un groupe alkyle ou un groupe alcényle en C₆-C₂₈, et dans laquelle R⁶⁰ et R⁶¹ représentent respectivement, indépendamment l'un de l'autre, un groupe alkyle en C₁-C₄, et dans laquelle α représente le nombre 0 ou 1, β et χ sont choisis respectivement, indépendamment l'un de l'autre, parmi les nombres entiers de 1 à 4, et dans laquelle Y représente un atome d'oxygène ou un atome d'azote, et dans laquelle X représente un anion compatible.

22. Agent selon l'une quelconque des revendications 1 à 21, **caractérisé en ce que**, en ce qui concerne le composé se fixant sur des surfaces de substrats dures et/ou souples, qui porte au moins une charge cationique, il s'agit d'une bétaïne de l'acide alkylamidoalcényldiméthylcarboxylique répondant à la formule (19) : 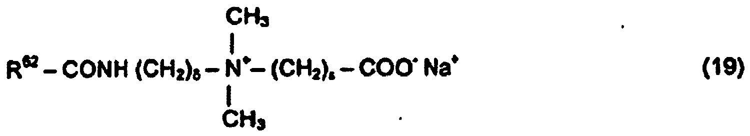 dans laquelle δ et ε représentent, indépendamment l'un de l'autre, des nombres entiers de 1 à 4 ; de préférence, δ est égal à 2 ou 3 et ε est égal à 2 ou 3, et dans laquelle R⁶² représente une chaîne alkyle en C₁₀-C₁₈ ou des mélanges desdites chaînes.

23. Agent selon l'une quelconque des revendications 1 à 22, **caractérisé en ce que**, en ce qui concerne le composé se fixant sur des surfaces de substrats dures et/ou souples, qui porte au moins une charge cationique, il s'agit d'un nitrile cationique répondant à la formule (14) : 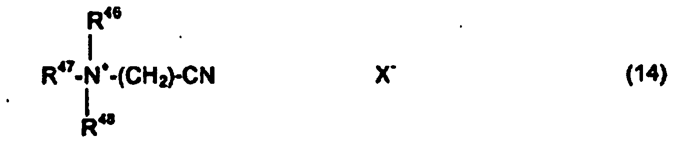 dans laquelle R⁴⁶ représente un atome d'hydrogène, un groupe CH₃, un résidu alkyle ou alcényle en C₂-C₂₄, un résidu alkyle ou alcényle en C₂-C₂₄ substitué avec au moins un substituant choisi parmi le groupe comprenant un atome de chlore, un atome de brome, un groupe -OH, un groupe -NH₂, un groupe -CN, ou un résidu alkylaryle ou alcénylaryle comprenant un groupe alkyle en C₁-C₂₄ et au moins un substituant supplémentaire sur le noyau aromatique, R⁴⁷ et R⁴⁸ sont choisis, indépendamment l'un de l'autre, parmi un groupe -CH₂-CN, un groupe -CH₃, un groupe -CH₂-CH₃, un groupe -CH₂-CH₂-CH₃, un groupe -CH(CH₃)-CH₃, un groupe -CH₂-OH, un groupe -CH₂-CH₂-OH, un groupe -CH(OH)-CH₃, un groupe -CH₂-CH₂-CH₂-OH, un groupe -CH₂-CH(OH)-CH₃, un groupe -CH(OH)-CH₂-CH₃, un groupe -(CH₂CH₂-O)ₙH où n est égal à 1, 2, 3, 4, 5 ou 6 et X représente un anion.

24. Agent selon l'une quelconque des revendications 1 à 23, **caractérisé en ce qu'**il renferme un nitrile cationique répondant à la formule (15) : 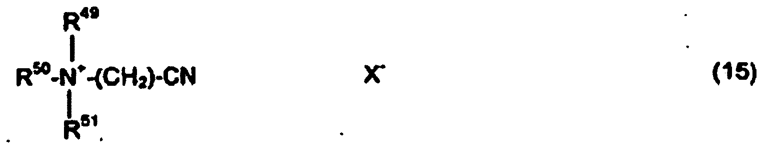 dans laquelle R⁴⁹, R⁵⁰ et R⁵¹ sont choisis indépendamment l'un de l'autre parmi un groupe -CH₃, un groupe -CH₂-CH₃, un groupe -CH₂-CH₂-CH₃, un groupe -CH(CH₃)-CH₃, R⁴⁹ pouvant en outre représenter également un atome d'hydrogène et X représente un anion, formule dans laquelle, à titre préférentiel, R⁵⁰ = R⁵¹ = -CH₃ et en particulier R⁴⁹ = R⁵⁰ = R⁵¹ = -CH₃, et des composés répondant aux formules (CH₃)₃N⁽⁺⁾CH₂-CN X⁻, (CH₃CH2)₃N⁽⁺⁾CH₂-CN X⁻, (CH₃CH₂CH₂)₃N⁽⁺⁾CH₂-CN X⁻, (CH₃CH(CH₃))₃N⁽⁺⁾CH₂-CN X⁻ ou (HO-CH₂-CH₂)₃N⁽⁺⁾CH₂-CN X⁻ étant particulièrement préférés.

25. Agent selon l'une quelconque des revendications 23 à 24, **caractérisé en ce que** X- représente un anion qui est choisi parmi le groupe comprenant un chlorure, un bromure, un iodure, un hydrogénosulfate, un méthosulfate, un laurylsulfate, un dodécylbenzènesulfonate, un p-toluènesulfonate (tosylate), un cumènesulfonate ou un xylènesulfonate, ou leurs mélanges.

26. Agent selon l'une quelconque des revendications 1 à 25, **caractérisé en ce que** l'agent est présent sous forme solide, sous forme dispersée, sous forme pulvérulente, sous forme granulaire ou sous forme comprimée, avec cette mesure que, lorsqu'il est présent sous forme comprimée, il est présent en particulier sous la forme d'un comprimé qui est constitué par une phase unique ou par plusieurs phases.

27. Agent selon l'une quelconque des revendications 1 à 25, **caractérisé en ce que** l'agent est présent sous la forme d'un gel ou sous forme liquide, en particulier sous forme émulsifiée, et contient de préférence un ou plusieurs solvants jusqu'à concurrence de 95 % en poids, de manière avantageuse à concurrence de 20 à 90 % en poids et de manière particulièrement préférée à concurrence de 50 à 80 % en poids.

28. Agent selon l'une quelconque des revendications 1 à 27, **caractérisé en ce qu'**il contient au moins une substance supplémentaire que contiennent habituellement des agents de lavage ou de nettoyage, de préférence une substance choisie parmi le groupe comprenant des agents tensioactifs, des builders (des builders organiques et inorganiques), des agents de blanchiment, des activateurs du blanchiment, des stabilisateurs du blanchiment, des catalyseurs du blanchiment, des enzymes, des polymères spéciaux (par exemple ceux possédant des propriétés de cobuilders), des inhibiteurs du grisaillement, des azurants optiques, des substances de protection contre le rayonnement ultraviolet, des agents repoussant les saletés, des électrolytes, des colorants, des fragrances, des substances aromatisantes, des porteurs de parfums, des agents de réglage du pH, des formateurs de complexes, des agents fluorescents, des inhibiteurs de mousse, des agents antifroissement, des antioxydants, des composés d'ammonium quaternaires, des agents antistatiques, des agents facilitant le repassage, des agents absorbant le rayonnement ultraviolet, des agents antiredéposition, des germicides, des substances actives antimicrobiennes, des fongicides, des régulateurs de la viscosité, des agents conférant un reflet nacré, des inhibiteurs du transfert de couleurs, des agents empêchant le rétrécissement, des inhibiteurs de la corrosion, des conservateurs, des adoucissants, des assouplissants, des hydrolysats de protéines, des agents d'imprégnation et rendant hydrophobe, des hydrotropes, des huiles de silicone ainsi que des agents de gonflement et des agents antiglissement.

29. Utilisation d'un agent selon l'une quelconque des revendications précédentes 1 à 28, à titre de formulation cosmétique.

30. Utilisation d'un agent selon l'une quelconque des revendications précédentes 1 à 28, à titre d'agent de traitement des textiles, en particulier à titre d'agent de lavage ou d'agent de traitement ultérieur ou d'agent adoucissant ou à titre d'assouplissant.

31. Utilisation d'un agent selon l'une quelconque des revendications précédentes 1 à 28, pour la revitalisation de fibres kératiniques.

32. Procédé pour le traitement de surfaces de substrats dures et/ou souples, **caractérisé en ce qu'**on applique une quantité efficace d'un agent selon l'une quelconque des revendications 1 à 28, en utilisant un atomiseur sur le substrat à traiter, avec cette mesure que l'agent est présent sous forme liquide, en particulier sous forme émulsifiée.

33. Utilisation d'un agent selon l'une quelconque des revendications précédentes 1 à 28, pour le nettoyage et/ou l'entretien de surfaces dures.

34. Produit contenant un agent selon l'une quelconque des revendications 1 à 28 et un atomiseur.

35. Substrat de conditionnement **caractérisé en ce qu'**il s'agit d'un substrat imprégné et/ou enduit avec un agent selon l'une quelconque des revendications 1 à 28.

36. Substrat de conditionnement selon la revendication 35, **caractérisé en ce que** le substrat est constitué d'un non-tissé, en particulier d'un non-tissé à base de viscose.

37. Substrat de conditionnement selon l'une quelconque des revendications 35 à 36, **caractérisé en ce que** le substrat présente un grammage entre 20 et 1000 g/m², en particulier de 30 à 500 g/m².

38. Substrat de conditionnement selon l'une quelconque des revendications 35 à 37, **caractérisé en ce que** le substrat présente une dimension de 0,2 à 0,005 m².

39. Procédé pour le conditionnement des textiles, **caractérisé en ce que** l'on met en oeuvre un ou plusieurs substrats de conditionnement selon l'une quelconque des revendications 35 à 38 dans un processus de séchage des textiles.

40. Utilisation d'un composé se fixant sur des surfaces de substrats dures et/ou souples, qui porte au moins une charge cationique, pour la fixation d'un oligomère, d'un polymère ou d'un copolymère qui contient, à raison d'au moins une fois, l'élément de structure ci-après répondant à la formule (1), sur des surfaces de substrats durs et/ou souples : 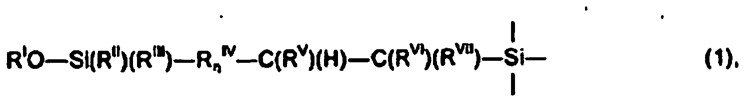 dans laquelle R^{II}, R^{III} représentent, indépendamment l'un de l'autre, respectivement un résidu d'hydrocarbure aliphatique ou aromatique, à chaîne droite ou ramifiée, saturé ou insaturé, substitué ou non substitué, qui peut contenir respectivement des hétéroatomes tels qu'un atome d'oxygène, un atome d'azote, un atome de soufre ou un atome d'halogène ou analogue, et dans laquelle R_{η}^{IV} représente un membre faisant pont à base de carbone qui représente un résidu d'hydrocarbure aliphatique ou aromatique, à chaîne droite ou ramifiée, saturé ou insaturé, substitué ou non substitué, qui peut contenir respectivement des hétéroatomes tels qu'un atome d'oxygène, un atome d'azote, un atome de soufre ou un atome d'halogène ou analogue, le numéro d'ordre η s'élevant de 0 à 10, et dans laquelle R^{V}, R^{VI}, R^{VII} représentent, indépendamment l'un de l'autre, respectivement un atome d'hydrogène ou un résidu d'hydrocarbure aliphatique ou aromatique, à chaîne droite ou ramifiée, saturé ou insaturé, substitué ou non substitué, qui peut contenir respectivement des hétéroatomes tels qu'un atome d'oxygène, un atome d'azote, un atome de soufre ou un atome d'halogène ou analogue, et dans laquelle le silicium terminal dans la formule (1) présente, à ses trois valences restantes, indépendamment l'une de l'autre, n'importe quel résidu de l'oligomère, du polymère ou du copolymère, et dans laquelle R^{I}O représente un résidu à savoir un groupe alcoxy de substance aromatisante et/ou un groupe alcoxy de biocide qui dérive de l'alcool de substance aromatisante et/ou de biocide R^{I}OH correspondant, ou bien R^{I}O représente un résidu qui dérive d'un ester, d'une cétone ou d'un aldéhyde énolisable de substance aromatisante et/ou de biocide.
